# EUROPEAN PATENT APPLICATION

(11) **EP 3 636 090 A1**
(43) Date of publication of application: **15.04.2020**
(21) Application number: 17912399.7
(22) Date of filing: 22.12.2017
(51) Int. Cl.: A41D 31/00, A41B 9/02, A41D 13/00, A61F 13/47

(54) **WATER REPELLENCY-IMPARTING FIBER ARTICLE**

(30) Priority: 09.06.2017 WO PCT/JP2017/021553
(71) Applicant: Kao Corporation, Chuo-ku Tokyo 103-8210 (JP)
(72) Inventor: TANAKA, Masahito, Tochigi 321-3497 (JP); KITO, Tetsuji, Wakayama-shi Wakayama 640-8404 (JP); OTANI, Kenichi, Tokyo 131-0044 (JP); NAKANISHI, Hirofumi, Tochigi 321-3497 (JP)
(74) Representative: Hoffmann Eitle
(86) International application number: PCT/JP2017/046254
(87) International publication number: WO 2018/225286

(57) **Abstract**

A water repellency-imparted fiber article (10) of the present invention includes: a fiber article (1) having a skin-facing surface (1a) and a non-skin-facing surface (1b); and a water repellency-imparting agent attached to the fiber article (1). The fiber article (1) contains a water-absorbent fiber and has water absorbency in terms of a water absorption time of 30 seconds or less as measured in accordance with the dropping method of JIS L-1907. The water repellency-imparted fiber article (10) has a non-skin-side water-repellent region (2) on the non-skin-facing surface (1b) of the fiber article (1), the non-skin-side water-repellent region (2) including a portion to which the water repellency-imparting agent is attached. The water repellency-imparted fiber article (10) has a water absorbing layer (11) in a section of the fiber article that overlaps the non-skin-side water-repellent region (2) in a plan view and on a side that is closer to the skin of a user than the non-skin-side water-repellent region (2), the water absorbing layer maintaining the water absorbency. A surface of the non-skin-side water-repellent region 2 has a water contact angle of 80 degrees or greater and a water absorption rate of 10 seconds or greater.

## Description

### Technical Field

The present invention relates to water repellency-imparted fiber articles obtained by applying water-repellent treatment to fiber articles, such as various items of clothing and incontinence pads, which are to be put on the skin when use.

### Background Art

Inner clothing typified by underwear is a typical example of a fiber article that is made mainly of fibers and is to be directly put on the skin when use. Also, a type of absorbent article is known which is attached to the inner side of underwear or the inner side of a diaper or a diaper cover and primarily absorbs urine. Such absorbent articles are also a kind of fiber article. Patent Literature 1 discloses, as a kind of such absorbent article, a male incontinence pad having a structure (leak-proof cuff) that stops a lateral flow of excreted urine so as to prevent urine from leaking out even if a large amount of urine is excreted at one time. It is required, for example, that a fiber article like this absorb a bodily fluid excreted from the body, such as sweat, urine, or blood, and be free from a wet feel and a sticky feel on a surface thereof that is in contact with the skin.

Patent Literature 2 discloses, as a fabric cloth that is free from a sticky feel even if a wearer wearing the fabric cloth sweats heavily, a fabric cloth having, in the surface thereof, a water-repellent sea (matrix) and a plurality of water-absorbent island (domains) therein, the area per single island and the ratio of the total area of the islands to the area of that surface being within respective specific ranges. The fabric cloth disclosed in Patent Literature 2 is produced by carrying out textile printing on the surface of a water-absorbent fabric cloth according to a known method, such as rotary screen printing, using various water repellents, such as fluorine or silicone water repellents.

Patent Literature 3 discloses that a woven or knitted fabric that is less likely to cause a wet feel and also has moisture absorbency can be obtained by subjecting a woven or knitted fabric containing a cotton fiber to alkali treatment to thereby remove cotton wax contained in the cotton fiber, and thereafter partially applying a water repellent to at least one of the front and back sides of the woven or knitted fabric. In Patent Literature 3, there is a description to the effect that the water repellent is preferably applied to only one side of the woven or knitted fabric that comes into contact with the skin, and that the application pattern of the water repellent is preferably a lattice pattern or a pattern such that polygons are successively arranged while sharing corners with each other.

Patent Literature 4 discloses, as a fabric cloth for preventing sweat stains that are caused as a result of sweating, a sweat-stain preventing fabric cloth made of a polyester fiber, wherein a water repellent is applied only the surface on one side of the fabric cloth while the surface on the other side has an uneven structure. The sweat-stain preventing fabric cloth disclosed in Patent Literature 4 is disposed when use such that the surface to which the water repellent is applied is arranged on an ambient air-side, and that the other surface, which has the uneven structure, is arranged on the skin side. In Patent Literature 4, there is a description to the effect that since the surface that comes into contact with the skin during use has the uneven structure, the area of contact between the skin and the fabric cloth decreases, resulting in a reduced sticky feel, and also that since sweat is diffused along depressions of the uneven structure, the drying rate increases, resulting in an enhancement in the sweat-stain preventing effect. Moreover, with regard to the water repellent, in Patent Literature 4, there is a description to the effect that applying the water repellent to the entire surface of the fabric cloth is preferable compared to applying it partially to the surface, in view of obtaining an excellent sweat-stain preventing effect.

Patent Literature 5 discloses a fluorochemical composition that can impart, to a fiber article, resistance against soil and stains caused by a bodily fluid such as sweat, or more specifically, oil repellency, water repellency, stain-release properties, stain resistance, and other properties. In Patent Literature 5, there is a description to the effect that, when this fluorochemical composition is applied to a fiber article to thereby form a coating thereon, the coating can be maintained even after the fiber article is washed a plurality of times.

### Citation List

### Patent Literature

Patent Literature 1: JP 2015-188714A

Patent Literature 2: JP 2012-126036A

Patent Literature 3: JP 2007-162150A

Patent Literature 4: JP 2011-226001A

Patent Literature 5: JP 2010-529263A

### Summary of Invention

The present invention provides a water repellency-imparted fiber article including: a fiber article having a skin-facing surface to be disposed on a side that is relatively close to the skin of a user during use and a non-skin-facing surface to be disposed on a side that is relatively away from the skin of the user, the fiber article containing a water-absorbent fiber and having water absorbency in terms of a water absorption time of 30 seconds or less as measured in accordance with the dropping method of JIS L-1907; and a water repellency-imparting agent attached to the fiber article.

The water repellency-imparted fiber article has a non-skin-side water-repellent region on the non-skin-facing surface of the fiber article, the non-skin-side water-repellent region including a portion to which the water repellency-imparting agent is attached.

The water repellency-imparted fiber article has a water absorbing layer in a section of the fiber article that overlaps the non-skin-side water-repellent region in a plan view and on a side that is closer to the skin of the user than the non-skin-side water-repellent region, the water absorbing layer maintaining the water absorbency.

A surface of the non-skin-side water-repellent region has a water contact angle of 80 degrees or greater and a water absorption rate of 10 seconds or greater.

Also, the present invention provides a method for producing the water repellency-imparted fiber article of the present invention, the method including applying a water repellency-imparting agent to a non-skin-facing surface of a fiber article having water absorbency in terms of a water absorption time of 30 seconds or less as measured in accordance with the dropping method of JIS L-1907.

### Brief Description of Drawings

[Fig. 1] Fig. 1 is a schematic view of a pair of water repellency-imparted underpants, which is a piece of inner clothing, of an embodiment of a water repellency-imparted fiber article of the present invention, and it shows a state in which the water repellency-imparted underpants is worn.
[Fig. 2] Fig. 2 is another schematic view of the water repellency-imparted underpants shown in Fig. 1, and it shows a state in which a pair of trousers, which is a piece of outer clothing, is worn on the underpants.
[Fig. 3] Fig. 3 is a schematic cross-sectional view of another embodiment of the water repellency-imparted fiber article of the present invention taken along a thickness direction thereof, and it shows a state in which the water repellency-imparted fiber article is used in combination with a piece of outer clothing.
[Fig. 4] Fig. 4(a) is a microscope photograph of a non-skin-side water-repellent region of a specific example of the water repellency-imparted fiber article of the present invention, and Fig. 4(b) is a microscope photograph of the opposite side to the side on which the non-skin-side water-repellent region shown in Fig. 4(a) is formed, that is, a skin-facing surface of the specific example.
[Fig. 5] Fig. 5 is an EDS image obtained by imaging, with an energy dispersive X-ray spectrometer (EDS), a cross section of a specific example of the water repellency-imparted fiber article of the present invention taken along the thickness direction thereof.
[Fig. 6] Fig. 6 is an EDS image of the specific example of the water repellency-imparted fiber article shown in Fig. 5 after washing, and it shows a state in which a water repellency-imparting agent has been removed through washing so that a non-skin-side water-repellent region has disappeared.

### Description of Embodiments

It is a distress to some people, particularly men, that urine is not completely expelled during urination, and that a small amount of urine leaks after urination, soiling their underpants. In particular, in older men, such a distress is more serious because of the following: due to urethral dilation or the like that accompanies aging, urine is more likely to remain in the urethra after urination, and the residual urine may be accidentally discharged from the penis, soiling the underpants. Such leakage of residual urine in the urethra is in an amount of about several to 10 mL, and often is so-called "after-dribble", which is a small amount, specifically about 1 mL or less, of urine leaks. Unlike incontinence, so-called "wetting", in which the underpants get wet through, such after-dribble does not necessarily require a changing of underpants, and partly because of this, some people do not regard such urine leakage as a problem that needs to be immediately solved. However, after-dribble may cause an unpleasant feeling due to wet underpants, and there also are cases where urine seeps even into outer clothing such as trousers, that is worn on inner clothing such as underpants, to soil the outer clothing, or where the odor diffuses into the surroundings. Thus, many people desire to find a countermeasure against these problems.

An example of a method for solving such problems arising from after-dribble, such as soiling clothes and causing odor, is use of a male incontinence pad such as that disclosed in Patent Literature 1. As for male incontinence pads, smaller pads for light incontinence, and the like have also been proposed in recent years that are intended to make it unlikely that other people notice that such a pad is worn. However, most men are reluctant to use an incontinence pad to deal with merely a small amount of urine leakage, such as after-dribble, and actually there is strong demand for solving the problems arising from after-dribble without needing to use an incontinence pad.

Moreover, although the techniques disclosed in Patent Literatures 2 and 3 increase the dry feel of a surface of a fiber article that comes into contact with the skin by applying a water repellent to that surface of the fiber article, the above-described soiling of clothes caused by after-dribble is left out of consideration, and these techniques are insufficient to deal with after-dribble. The sweat-stain preventing fabric cloth disclosed in Patent Literature 4 is effective to a certain extent in preventing sweat stains; however there is concern that air permeability will decrease, particularly due to a configuration in which the water repellent is attached to the entire surface of the fabric cloth, and, for example, when the fabric cloth is used as a constituent member of a wearable article, the fabric cloth may cause odor and skin problems such as dampness and rashes. Also, since the sweat-stain preventing fabric cloth disclosed in Patent Literature 4 is made of polyester fibers, the fabric cloth itself has poor water absorbency, and in terms of the wearing comfort as well, the fabric cloth is inferior to those made of water-absorbent fibers such as a natural cellulose fiber. Furthermore, if the fluorochemical composition disclosed in Patent Literature 5 is applied to a fiber article, such as underpants, to thereby make a portion of the fiber article hydrophobic, this may be effective in preventing soiling of clothes caused by after-dribble, but there is a risk that the fiber article will become excessively water-repellent, which results in that the water absorbency, flexibility, air permeability, and the like intrinsic to the fiber article may be reduced.

As described above, it is a distress to many people that a bodily fluid, such as a relatively small amount of urine or sweat, seeps into clothes, but the use of dedicated goods, such as incontinence pads and underarm pads, for dealing with this problem tends to be avoided. A technique has not yet been provided that can solve the above-described problems while using commonly-used clothing, such as underwear, as-is, without needing to use such dedicated goods.

Therefore, the present invention relates to providing a water repellency-imparted fiber article that can effectively prevent a bodily fluid excreted from the body from seeping into the clothing and thus becoming externally visible.

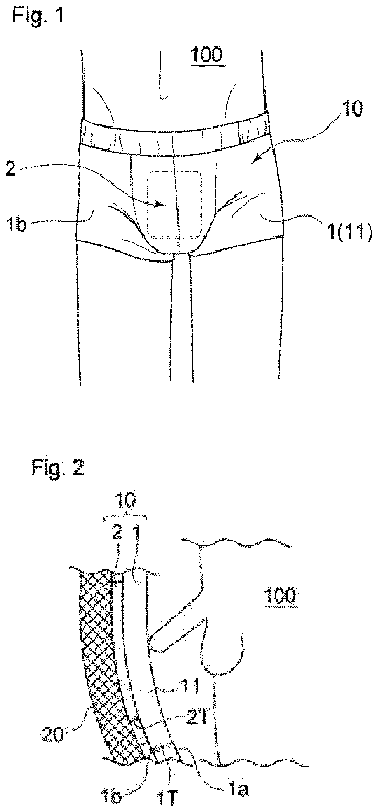

Hereinafter, the present invention will be described in detail. Fig. 1 is a schematic view of a pair of water repellency-imparted underpants 10, which is an embodiment of a water repellency-imparted fiber article of the present invention, when worn, and Fig. 2 is a schematic view showing a state in which a pair of trousers 20, which is a piece of outer clothing, is worn on the water repellency-imparted underpants 10. The pair of water repellency-imparted underpants 10 includes a pair of underpants 1, which is a fiber article (fiber article that has not yet been subjected to water repellency-imparting treatment) according to the present invention, as well as a water repellency-imparting agent (non-skin-side water-repellent region 2) attached to the underpants 1.

The underpants 1, which are an embodiment of the fiber article according to the present invention, are basically configured in the same manner as in common trunks-type underpants for men, which are pieces of inner clothing, and have a front body portion, a back body portion, and a crotch portion located therebetween, as well as a waist opening and a pair of leg openings. The underpants 1 are so-called front-closed-type trunks with no front opening, and a waist end portion thereof that defines the waist opening is elasticized by, for example, disposing a filamentous elastic member therein. The underpants 1 have a skin-facing surface 1a to be disposed on a side that is relatively close to the skin of a user during use, and a non-skin-facing surface 1b to be disposed on a side that is relatively away from the skin of the user. When the underpants 1 are worn as shown in Fig. 2, the skin-facing surface 1a is in contact with the skin of a wearer 100 of the underpants, and the non-skin-facing surface 1b is in contact with the trousers 20. The underpants 1 constitute the main body of the water repellency-imparted underpants 10, and the appearance, texture, wearing comfort, and the like of the water repellency-imparted underpants 10 are basically derived from the intrinsic performance of the underpants 1 that is the base of the water repellency-imparted underpants 10.

There is no particular limitation on the fiber article, which is the base of the water repellency-imparted fiber article of the present invention, and in the cases where underpants are concerned, not only trunks-type underpants, such as those shown in Figs. 1 and 2, in which the front body portion has a single-layer structure, but also, for example, so called briefs-type underpants in which the front body portion has a double structure can be used. In the case where the front body portion of the underpants 1 has such a multilayer structure, a surface (outer surface) of the outermost layer, of that multilayer structure, that is the farthest away from the skin of the wearer is the non-skin-facing surface 1b that comes into contact with the trousers 20. This concept of the skin-facing surface and the non-skin-facing surface with respect to the fiber article having a multilayer structure applies to fiber articles other than underpants.

The underpants 1, which are the fiber article that is the base of the water repellency-imparted underpants 10 serving as the water repellency-imparted fiber article, will be described in greater detail. The underpants 1 include a fiber sheet 11 that is made mainly of fibers including a water-absorbent fiber. The fiber sheet 11 constitutes the main body of the underpants 1, and the front body portion, the back body portion, and the crotch portion of the underpants 1 are formed by the fiber sheet 11. The skin-facing surface 1a of the underpants 1 is the skin-facing surface of the fiber sheet 11, and the non-skin-facing surface 1b of the underpants 1 is the non-skin-facing surface of the fiber sheet 11. The following description regarding the fiber sheet 11 applies to the underpants 1 (fiber article) as is, unless otherwise specified.

The fiber sheet 11 is made mainly of fibers including a water-absorbent fiber. The fiber content in the fiber sheet 11 is preferably 50 mass% or greater, or more preferably 70 mass% or greater, or may also be 100 mass%, that is, the fiber sheet 11 may consist of fibers. Fibers that are usually used in various fiber products including clothing can be used as the constituent fibers of the fiber sheet 11 without limitation. Any of natural fibers and chemical fibers may be used, and the fibers may be used singly or in combinations of two or more thereof.

The natural fibers may be plant fibers or animal fibers. Examples of the plant fibers include wood pulp such as softwood pulp and hardwood pulp, as well as non-wood fibers such as cotton and hemp. Examples of the animal fibers include silk and wool.

Examples of the chemical fibers include synthetic fibers made of resins, such as polyester fibers, acrylic fibers, modacrylic fibers, nylon fibers, vinylon fibers, polypropylene fibers, polyvinylchloride fibers, polyethylene fibers, vinylidene fibers, and polyurethane fibers; regenerated fibers such as rayon, polynosic, cupra, and Lyocell; semisynthetic fibers such as acetate and triacetate; and inorganic fibers such as glass fibers, metallic fibers, and carbon fibers.

The fiber sheet 11 contains a water-absorbent fiber. In the present invention, a "water-absorbent fiber" literally refers to a fiber that has water absorbency, or more specifically, a fiber that has an official moisture regain of 5% or greater. The water-absorbent fiber may be a fiber that intrinsically has water absorbency, or may be a fiber that is obtained by processing a fiber that intrinsically does not have water absorbency to impart water absorbency thereto. The above-described natural fibers, as well as the regenerated fibers and the semisynthetic fibers of the above-described chemical fibers can be used as the water-absorbent fiber in the present invention.

Moreover, examples of the synthetic fibers that can be used as the water-absorbent fiber in the present invention include intrinsically hydrophilic synthetic fibers, such as polyvinyl alcohol fibers and polyacrylonitrile fibers; and fibers (hydrophilized fibers) obtained by subjecting intrinsically hydrophobic synthetic fibers, such as polyethylene terephthalate fibers, polyethylene fibers, polypropylene fibers, and polyester fibers, to hydrophilization treatment. Examples of the hydrophilized fibers include synthetic fibers into which a hydrophilizing agent is incorporated, synthetic fibers with a hydrophilizing agent attached to a surface thereof, and synthetic fibers subjected to plasma treatment. There is no particular limitation on the hydrophilizing agent, as long as the hydrophilizing agent is a common hydrophilizing agent for use in various fiber products including clothing. The synthetic fiber may be a single-component fiber made of a single type of synthetic resin or a polymer blend of two or more types of synthetic resins, or may be a composite fiber. As used herein, the composite fiber refer to a synthetic fiber which is obtained by combining two or more types of synthetic resins having different components in a spinneret and simultaneously spinning these synthetic resins therethrough, and in which the plurality of components individually extending continuously in a fiber length direction adhere to one another within each single fiber. The form of the composite fibers may be, but not limited to, a core-sheath type, a side-by-side type, or the like.

The form of the fiber sheet 11 is not limited, and may be, for example, a woven fabric (woven cloth), a knitted fabric, a nonwoven fabric, or paper. In the case where the fiber sheet 11 is a woven fabric or a knitted fabric, there is no particular limitation on the weave or the knitting pattern. Examples of the woven fabric include woven fabrics woven in a plain weave, a twill weave, a satin weave, or the like. Examples of the knitted fabric include weft-knitted fabrics such as flat knits, rib knits, purl knits, and interlock knits; and warp-knitted fabrics such as single tricot knits, single cord knits, half tricot knits, plain tricot knits, and queens cord knits. Examples of the nonwoven fabric include through-air bonded nonwoven fabrics, spunbonded nonwoven fabrics, spunlaced nonwoven fabrics, meltblown nonwoven fabrics, resin-bonded nonwoven fabrics, and needlepunched nonwoven fabrics.

The fiber sheet 11 may have a single-layer structure, or a laminated structure in which a plurality of sheets having a single-layer structure are laminated. Examples of the nonwoven fabric having a laminated structure include a spunbonded-spunbonded laminated nonwoven fabric (SS nonwoven fabric), a spunbonded-spunbonded-spunbonded laminated nonwoven fabric (SSS nonwoven fabric), a spunbonded-meltblown-spunbonded laminated nonwoven fabric (SMS nonwoven fabric), and a spunbonded-meltblown-meltblown-spunbonded nonwoven fabric (SMMS nonwoven fabric).

The fiber sheet 11 has water absorbency in terms of a water absorption time of 30 seconds or less, preferably 20 seconds or less, or more preferably 15 seconds or less as measured in accordance with the dropping method of JIS L-1907. The water repellency-imparted underpants 10 (underpants 1) include the fiber sheet 11 having the above-described water absorbency, and therefore can absorb water and various aqueous liquids. For example, the water repellency-imparted underpants 10 (underpants 1) can absorb sweat, urine, and blood as bodily fluids excreted from the body.

In view of securing the above-described water absorbency of the fiber sheet 11, the fiber sheet 11 preferably contains the water-absorbent fiber in a certain amount or greater. Specifically, the percentage (the occupancy ratio) of the water-absorbent fiber to all the constituent fibers of the fiber sheet 11 is preferably 50 mass% or greater, or more preferably 70 mass% or greater, or may also be 100 mass%, that is, all of the constituent fibers may be water-absorbent fibers.

The fiber sheet 11 may contain both a natural fiber and a synthetic fiber as its constituent fibers. As used herein, the synthetic fiber encompasses both a water-absorbent fiber and a fiber that is not water-absorbent (non-water-absorbent fiber). A feature of the fiber sheet 11 having such a mixed form of a natural fiber and a synthetic fiber is that dimensional shrinkage and shape collapse during wetting and after repeated wetting and drying (after repeated use) are less likely to occur. In view of enhancing such a feature of the mixed form of a natural fiber and a synthetic fiber, the mass ratio between the natural fiber and the synthetic fiber contained in the fiber sheet 11 having the mixed form, natural fiber/synthetic fiber, is preferably 1/9 to 9/1, and more preferably 2/8 to 8/2.

Alternatively, the fiber sheet 11 may contain only a natural fiber as its constituent fiber. A feature of the fiber sheet 11 having such a single-component fiber form is that the fiber sheet 11 exhibits excellent repeated water absorbency and is less irritating to the skin. As the fiber sheet 11 having a single-component fiber form, woven cloths or knitted cloths made of a cotton fiber are especially preferable that are produced by spinning a cotton fiber as the natural fiber and weaving or knitting the cotton fiber in any of various manners.

One of main features of the water repellency-imparted underpants 10 as an embodiment of the water repellency-imparted fiber article of the present invention is as follows. As shown in Figs. 1 and 2, the water repellency-imparted underpants 10 have a non-skin-side water-repellent region 2 on the outer surface (i.e., the non-skin-facing surface 1b) of the underpants 1 (fiber article), the non-skin-side water-repellent region 2 including a portion to which the water repellency-imparting agent is attached; and the water repellency-imparted underpants 10 also have a water absorbing layer in a section of the underpants 1 that overlaps the non-skin-side water-repellent region 2 in a plan view and on the side that is closer to the skin of a wearer (indicated by the reference numeral 100 in the drawings) than the non-skin-side water-repellent region 2, the water absorbing layer maintaining the above-described water absorbency, that is, the "water absorbency in terms of a water absorption time of 30 seconds or less as measured in accordance with the dropping method of JIS-L-1907". It is the fiber sheet 11 that functions as the water absorbing layer in the water repellency-imparted underpants 10. In short, in the water repellency-imparted underpants 10, the fiber sheet 11, which serves as the water absorbing layer, and the non-skin-side water-repellent region 2 are provided in that order from the side closer to the skin of the wearer of the underpants in the thickness direction thereof.

In the water repellency-imparted underpants 10, the non-skin-side water-repellent region 2 functions as a bodily fluid barrier layer for preventing a liquid from seeping out of the fiber sheet 11, which is the water absorbing layer. For example, in a state in which the trousers 20 as the outer clothing are worn on the water repellency-imparted underpants 10 as the inner clothing as shown in Fig. 2, if urine is excreted in an amount as small as about 1 mL or less from an excretion part (penis) of the wearer 100 of the underpants (in other words, so-called "after-dribble" occurs), there is concern that the excreted urine will pass through the fiber sheet 11 as the water absorbing layer in the thickness direction thereof and reach the trousers 20, problems arising such as making a urine stain (wet stain) that is externally visible on the trousers 20 and diffusing the odor of urine into the surroundings. However, in the water repellency-imparted underpants 10, the non-skin-side water-repellent region 2 functions as the bodily fluid barrier layer, or more specifically, a stain barrier layer, thereby preventing the urine from transferring toward the trousers 20, and therefore, the above-described problems can be prevented.

Moreover, even though the water repellency-imparted underpants 10 adopt the measure to prevent bodily fluid seepage, specifically, the formation of the non-skin-side water-repellent region 2, the basic configuration of the water repellency-imparted underpants 10 is substantially unchanged from the configuration of the underpants 1 (fiber article), which is the base thereof. Therefore, while the measure to prevent bodily fluid seepage is adopted, this is unlikely to be noticed by other people, and it is possible to use the water repellency-imparted underpants 10 with the same feeling as when wearing common underwear. Moreover, the non-skin-side water-repellent region 2 is formed on only the non-skin-facing surface 1b of the underpants 1, and the influences of the formation of the non-skin-side water-repellent region 2 on the underpants 1 are minimized. Therefore, the basic performance, such as the appearance, wearing comfort, and water absorbency, of the water repellency-imparted underpants 10 is by no means inferior to, and in some cases may even be superior to, the intrinsic performance of the underpants 1, and the water repellency-imparted underpants 10 also have excellent texture, usage feel, and the like.

In the water repellency-imparted underpants 10, the non-skin-side water-repellent region 2 is formed partially on the non-skin-facing surface 1b (non-skin-facing surface of the fiber sheet 11) of the underpants 1. Specifically, as shown in Figs. 1 and 2, the non-skin-side water-repellent region 2 is formed in only a section of the front body portion of the underpants 1 that faces the excretion part (penis) of the wearer 100 of the underpants, and has a substantially quadrangular shape in a plan view.

In the water repellency-imparted fiber article of the present invention, the non-skin-side water-repellent region serving as a stain barrier material against bodily fluids and the like is formed on at least the non-skin-facing surface of the fiber article, but it is not particularly limited in what area the non-skin-side water-repellent region is formed. In short, there is no particularly limitation on in what area the non-skin-side water-repellent region is formed as long as the non-skin-side water-repellent region is formed so as to include a section where prevention of liquid seepage is desired. For example, in the water repellency-imparted underpants 10, the non-skin-side water-repellent region 2 may be formed over the entire area of the non-skin-facing surface 1b in the front body portion of the underpants 1. The section where the non-skin-side water-repellent region 2 is formed on the non-skin-facing surface 1b need not be a portion that corresponds to the pubic region of the wearer (central portion of the underpants 1 in a front view) as shown in Fig. 1, and it can be anywhere, and for example, can correspond to a section where the wearer is especially anxious about the bodily fluid seepage. Specifically, for example, when an imaginary center line that bisects the body of the wearer in the width direction of the wearer while extending in the height direction of the wearer is used as a reference line, the non-skin-side water-repellent region 2 may be formed to the right or the left of the reference line.

In the water repellency-imparted underpants 10, the surface of the non-skin-side water-repellent region 2 has a water contact angle of 80 degrees or greater, preferably 85 degrees or greater, and more preferably 90 degrees or greater. The contact angle is an index of the degree of hydrophobicity of the surface of the non-skin-side water-repellent region 2. The greater the numerical value of the contact angle, the higher the hydrophobicity (the lower the hydrophilicity), and the smaller the numerical value of the contact angle, the lower the hydrophobicity (the higher the hydrophilicity). If the water contact angle on the surface of the non-skin-side water-repellent region 2 is less than 80 degrees, it is not possible to prevent liquid from seeping out of the water absorbing layer (fiber sheet 11), and thus problems, specifically, a bodily fluid excreted from the body seeping into the clothing and becoming externally visible, cannot be prevented from arising.

Conventionally, in the context of imparting water repellency to an object (fiber article), it is common that "water repellency" to be imparted is greater than 90 degrees in terms of the water contact angle as an index. However, the conditions that liquid transfer from the inner clothing side, such as a piece of underwear, to the outer clothing side, such as a pair of trousers, can be prevented, and that liquid can be confined to the inside of the inner clothing suffice for the technical idea of the present invention. For this purpose, the condition that a sufficiently lower degree of hydrophilicity (higher degree of hydrophobicity) than the hydrophilicity of the outer clothing is imparted to the inner clothing suffices. These findings have been obtained after various studies conducted by the inventors of the present invention, and the above-described preferable range (80 degrees or greater) of the water contact angle on the surface of the non-skin-side water-repellent region 2 is based on these findings. The contact angle is measured in accordance with the following method.

Method for Measuring Contact Angle (Contact Angle on Sheet Surface)

A measurement sample is cut from a fiber article to be measured, the measurement sample having a quadrangular shape in a plan view with a size of 150 mm in the longitudinal direction (MD direction) and 70 mm in the lateral direction (CD direction). The measurement sample is allowed to stand for twenty-four hours in a predetermined measurement environment for conditioning. With regard to the measurement environment, the temperature is set at 23 ± 2°C, and the relative humidity is set at 50 ± 5% RH. A drop of ion exchanged water is attached to a surface (non-skin-side water-repellent region) of the measurement sample on which the contact angle is to be measured. The image of the drop is recorded, and the contact angle is measured on the recorded image. More specifically, a microscope VHX-1000 manufactured by Keyence Corporation is used as the measuring apparatus, and a medium-magnification zoom lens is attached to the microscope in a state in which the zoom lens is tilted to 90°. The measurement sample is placed on a measurement stage of the measuring apparatus such that the surface to be measured faces upward and can be observed in the CD direction of the measurement sample. Then, a drop of 3 µL of ion exchanged water is attached to the surface to be measured, of the measurement sample placed on the measurement stage, and images of that drop are recorded and captured into the measuring apparatus. At this time, the images are captured in 3 seconds. Out of the plurality of recorded images, ten images in which both ends or one end of the drop in the CD direction is clear are selected, and for each of the ten images, the contact angle of the drop is measured based on a reference plane. The average value of the found contact angle values is taken as the contact angle on that measured surface (surface of the non-skin-side water-repellent region) of the fiber article of interest.

In the above-described method for measuring the contact angle, the surface unevenness may be significant depending on the weave structure of the fabric of the fiber article to be measured, and this may make it impossible to define the reference plane in magnified observation. In that case, the measurement can be performed in the same manner as in the method described above, except that the amount of drop of ion exchanged water attached is changed from 3 µL to 0.1 mL, the drop being attached in a stationary manner using a Komagome pipette, and that images are captured unmagnified (1/1).

Moreover, instead of the above-described "water contact angle", the "water repellency angle", which is measured using the following method, can also be used as an index of the degree of hydrophobicity of the surface of the non-skin-side water-repellent region. The term "water repellency angle" as used herein,may also be referred to as the "sliding angle". Depending on, for example, the type of the fiber article having the non-skin-side water-repellent region, the water repellency angle may be measured in a more practical condition than the water contact angle, and can more properly indicate the degree of hydrophobicity of the surface of the non-skin-side water-repellent region.

### Method for Measuring Water Repellency Angle (Sliding Angle)

A measurement sample is cut from a fiber article to be measured, the measurement sample having a quadrangular shape in a plan view with a size of 150 mm in the longitudinal direction (MD direction) and 70 mm in the lateral direction (CD direction). The measurement sample is allowed to stand for twenty-four hours in a predetermined measurement environment for conditioning. With regard to the measurement environment, the temperature is set at 23 ± 2°C and the relative humidity is set at 50 ± 5% RH. The measurement sample is fixed to one side of a 1-mm thick acrylic plate that has smooth surfaces. When fixing the measurement sample to the acrylic plate, it is preferable to extend the four sides of the measurement sample and fix the four sides with pressure-sensitive adhesive tape or the like so as not to form wrinkles on the measurement sample.

The acrylic plate to which the measurement sample is fixed is inclined relative to a horizontal plane, with the surface to which the measurement sample is fixed facing upward, and is fixed in this state. A drop of 0.1 mL of ion exchanged water is gently dropped onto the measurement sample from a position that is 10 mm above the measurement sample. The dropping operation in this manner is performed a plurality of times while the angle between the acrylic plate and the horizontal plane, that is, "the inclination angle of the measurement sample" is changed as appropriate. The inclination angle of the measurement sample when the drop is not absorbed by the measurement sample at the dropped position and rolls down the inclined surface of the measurement sample for a distance of 30 mm or greater is taken as the water repellency angle (sliding angle) of the measured surface (surface of the non-skin-side water-repellent region) of the fiber article of interest.

Usually, the water repellency angle (sliding angle) is within a range of greater than 0 degrees and less than 90 degrees, and as the value of the water repellency angle is smaller, it can be determined that the measured surface (surface of the non-skin-side water-repellent region) is more water-repellent, and therefore that the measured surface easily repels the drop. Thus, when the surface of the non-skin-side water-repellent region in the present invention is a water-repellent surface having a relatively low water repellency angle, seepage of a liquid to the outer clothing side, such as a pair of trousers, can be effectively prevented. From this point of view, in the water repellency-imparted underpants 10, a water repellency angle of the surface of the non-skin-side water-repellent region 2 on the non-skin-facing surface 1b is 45 degrees or less, preferably 40 degrees or less, and more preferably 30 degrees or less.

With regard to the skin-facing surface 1a of the underpants 1, the fiber sheet 11, which serves as the water absorbing layer maintaining the above-described water absorbency (water absorbency in terms of a water absorption time of 30 seconds or less as measured in accordance with the dropping method of JIS L-1907), needs to be provided in a section that overlaps the non-skin-side water-repellent region 2 in a plan view and on a side that is closer to the skin of the user than the non-skin-side water-repellent region 2 as described above, and a comfortable usage feel without a wet feel is required in contrast to the non-skin-facing surface 1b. Taking these requirements into account, it is not preferable that the skin-facing surface 1a of the underpants 1 be excessively water-repellent. From this point of view, the skin-facing surface 1a has a water contact angle of preferably 60 degrees or less, and more preferably 40 degrees or less. The skin-facing surface 1a is even more preferably configured such that, when a drop of ion exchanged water is attached to the skin-facing surface 1a as a surface to be measured in the above-described measurement method, the drop is absorbed into the underpants 1 via the skin-facing surface 1a almost simultaneously (instantly) with the attachment of the drop thereto. In other words, the skin-facing surface 1a of the underpants 1 even more preferably has high water absorbency such that the measurement of the contact angle of ion exchanged water is impossible. From the same point of view, the skin-facing surface 1a has a water repellency angle (sliding angle) of preferably 50 degrees or greater, and more preferably 55 degrees or greater. The skin-facing surface 1a is even more preferably configured such that, irrespective of the water repellency angle, a drop dropped onto the measurement sample in the above-described measurement method is quickly absorbed at the dropped position without rolling down the inclined surface.

In the fiber article, if a portion of the skin-facing surface thereof that is located on the opposite side to the non-skin-side water-repellent region (portion where the water repellency-imparting agent is attached) has a water contact angle of greater than 60 degrees or a water repellency angle of less than 50 degrees, or has high water absorbency such that the measurement of contact angle is impossible as described above, it can be determined that this fiber article does not have a water absorbing layer which maintains the above-described water absorbency and is located in a section that overlaps the non-skin-side water-repellent region in a plan view and on a side that is closer to the skin of the user than the non-skin-side water-repellent region.

As described above, the water contact angle or the water repellency angle (sliding angle) may be used as the index of the degree of hydrophobicity of the surface of the non-skin-side water-repellent region, or further, the skin-facing surface or the non-skin-facing surface of the fiber article. For the water repellency-imparted underpants 10, it is preferable that the surface of the non-skin-side water-repellent region 2 have a water contact angle within the above-described preferable range and also has a water repellency angle within the above-described preferable range.

Moreover, in addition to a water contact angle of 80 degrees or greater or a water repellency angle of 45 degrees or less, the surface of the non-skin-side water-repellent region 2 provides a water absorption rate of 10 seconds or greater, preferably 25 seconds or greater, and more preferably 40 seconds or greater. The water absorption rate as used herein is expressed as the time taken for a predetermined amount of water to be absorbed (i.e., water absorption time), and it is determined that as the numerical value of the water absorption rate is greater (the water absorption time is longer), the water absorption rate is slower. When the surface of the non-skin-side water-repellent region 2 has a water absorption rate of 10 seconds or greater, the amount of water absorbed on the non-skin-facing surface 1b side, on which the non-skin-side water-repellent region 2 has been provided, of the underpants 1 is relatively small, compared with that on the skin-facing surface 1a side, which is located on the opposite side to the non-skin-facing surface 1b side. Therefore, a liquid that is absorbed on the skin-facing surface 1a side is effectively suppressed from transferring to the non-skin-facing surface 1b side, and consequently seepage of the liquid into the trousers 20 can be even more effectively prevented. If the water absorption rate of the surface of the non-skin-side water-repellent region 2 is less than 10 seconds, seepage of a liquid out of the water absorbing layer (fiber sheet 11) cannot be prevented, and therefore, it is not possible to prevent problems, specifically, a bodily fluid excreted from the body seeping into the clothing and becoming externally visible. The water absorption rate is measured in accordance with the method described later.

Moreover, in view of even further increasing the effect of preventing a liquid from seeping into the trousers 20, it is preferable that the water absorption rate (water absorption time) of the surface of the non-skin-side water-repellent region 2 is slower, by 5 seconds or longer, than that of the surface of a portion of the skin-facing surface 1a of the underpants 1 that is located on the opposite side to the non-skin-side water-repellent region 2. Moreover, in view of achieving both the effective prevention of liquid seepage into the trousers 20 and the air permeability, the water absorption rate is preferably 600 seconds or less, and more preferably 300 seconds or less.

### Method for Measuring Water Absorption Rate

Five measurement samples are cut from a fiber article to be measured, the measurement samples each having a quadrangular shape in a plan view with a size of 200 mm in the longitudinal direction (MD direction) and 200 mm in the lateral direction (CD direction). The measurement samples are allowed to stand for twenty-four hours in a predetermined measurement environment for conditioning. With regard to the measurement environment, the temperature is set at 23 ± 2°C, and the relative humidity is set at 50 ± 5% RH. The measurement of water absorption rate is performed on these measurement samples in accordance with the method specified in JIS L1907, provided that the height from a surface to be measured of a measurement sample to the tip of a burette is changed to 5 mm. The surface to be measured refers to a surface of that measurement sample on which the water absorption rate is to be measured, and is either a skin-facing surface or a non-skin-facing surface (surface of the non-skin-side water-repellent region).

The water contact angle and the water absorption rate of the surface of the non-skin-side water-repellent region 2 can be adjusted by appropriately adjusting the type, the amount attached, the attachment area, and the like of the water repellency-imparting agent that forms the non-skin-side water-repellent region 2. Moreover, as will be described later, the non-skin-side water-repellent region 2 is formed by applying the water repellency-imparting agent to the non-skin-facing surface 1b of the underpants 1 (fiber article) that is the base of the water repellency-imparted underpants 10, and the water contact angle and the water absorption rate of the surface of the non-skin-side water-repellent region 2 can also be adjusted by devising application conditions including the viscosity of the water repellency-imparting agent and the pressure applied during application.

The amount (areal weight) of the water repellency-imparting agent that is attached to the non-skin-side water-repellent region 2 is preferably 0.02 g/m² or greater, and more preferably 0.05 g/m² or greater, and is preferably 1.5 g/m² or less, and more preferably 1.2 g/m² or less.

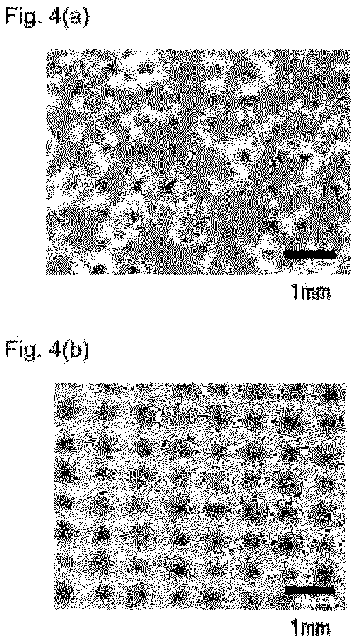

The non-skin-side water-repellent region 2 is a region including the "portion where the water repellency-imparting agent is attached" on the non-skin-facing surface 1b of the underpants 1. The non-skin-side water-repellent region 2 may have the following configuration i) or ii): i) the water repellency-imparting agent is applied continuously in a direction in which the plane of the non-skin-facing surface 1b extends, and no portions to which the water repellency-imparting agent is not attached are present; or ii) both a portion to which the water repellency-imparting agent is attached and a portion to which the water repellency-imparting agent is not attached are present. In the configuration ii), the water repellency-imparting agent is discontinuously present on the non-skin-facing surface 1b, that is, a plurality of portions to which the water repellency-imparting agent is attached are intermittently present; however the plurality of portions to which the water repellency-imparting agent is attached are gathered to at least an extent such that those portions can be regarded as a single integrated region, that is, the non-skin-side water-repellent region 2, by an observer. Fig. 4(a) is a microscope photograph (at a magnification of 50) of a specific example of a non-skin-side water-repellent region that has the configuration ii). In the non-skin-side water-repellent region shown in Fig. 4(a), it can be seen that both the portions to which the water repellency-imparting agent is attached (non-white portions) and the portions to which the water repellency-imparting agent is not attached (portions where the surface of the fiber article is exposed; white portions) are present.

As a result of applying the water repellency-imparting agent to the non-skin-facing surface 1b of the underpants 1 to thereby form the non-skin-side water-repellent region 2 thereon, a bodily fluid excreted from the body is prevented from seeping into clothing and becoming externally visible, but on the other hand there is concern that the air permeability and the flexibility of a section where the non-skin-side water-repellent region 2 is formed will be reduced. In this context, if both the portions to which the water repellency-imparting agent is attached and the portions to which the water repellency-imparting agent is not attached intermingle in the non-skin-side water-repellent region 2 as is in case of the above-described configuration ii) (see Fig. 4(a)), reductions in air permeability and flexibility caused by the application of the water repellency-imparting agent are suppressed, and therefore, the air permeability and the flexibility intrinsic to the underpants 1 can be substantially maintained. To form a non-skin-side water-repellent region 2 in which both a portion to which the water repellency-imparting agent is attached and a portion to which the water repellency-imparting agent is not attached intermingle, the water repellency-imparting agent can be intermittently attached to the non-skin-facing surface 1b of the underpants 1, for example, during the production of the water repellency-imparted underpants 10. In order to apply the water repellency-imparting agent intermittently, a water repellency-imparting agent may be sprayed onto the non-skin-facing surface 1b with a contactless application means to apply the repellency-imparting agent such as a spray; a water repellency-imparting agent may be applied according to a roll-on method, which will be described later; or a water repellency-imparting agent that is solidified into a stick-like shape may be brought into direct contact with the non-skin-facing surface 1b to thereby apply the water repellency-imparting agent thereto. A mixed state as shown in Fig. 4(a), in which both a portion to which the water repellency-imparting agent is attached and a portion to which the water repellency-imparting agent is not attached intermingle, can be obtained by any of these methods.

Fig. 4(b) is a microscope photograph (at a magnification of 50) of the opposite side to the side on which the non-skin-side water-repellent region is formed (side to which the water repellency-imparting agent is applied) shown in Fig. 4(a); that is, the microscope photograph shows the skin-facing surface (the side to which the water repellency-imparting agent is not applied) of the specific example of the water repellency-imparted fiber article of the present invention. In Fig. 4(a), the presence of the water repellency-imparting agent (non-white portions in the photograph) can be confirmed. However, in Fig. 4(b), the presence of the water repellency-imparting agent cannot be confirmed, and merely the original surface (skin-facing surface) of the fiber article is extending. The fact that the presence of the water repellency-imparting agent cannot be confirmed in Fig. 4(b) means that in the case where the non-skin-side water-repellent region 2 is formed by applying the water repellency-imparting agent to the non-skin-facing surface 1b of the underpants 1 by, for example, the roll-on method described later, it is unlikely that the applied water repellency-imparting agent penetrates the underpants 1 in the thickness direction thereof and appears on the skin-facing surface 1a. Thus, it can be said that there is a possibility that a "state in which the amount of water repellency-imparting agent attached decreases from the non-skin-facing surface of the fiber article toward the skin-facing surface thereof' will be provided by applying the water repellency-imparting agent to the non-skin-facing surface of the fiber article. This state will be described later.

In a 50-mm-square region (unit region) arbitrarily selected from the non-skin-side water-repellent region 2, the percentage of the total area of the portions to which the water repellency-imparting agent is attached (the occupancy ratio of portions to which the water repellency imparting agent is attached) to the whole area of the 50-mm-square region is preferably 10% or greater, and more preferably 15% or greater, and is preferably 80% or less, and more preferably 60% or less. The non-skin-side water-repellent region 2 in which the occupancy ratio of portions to which the water repellency imparting agent is attached is within the above-described range has the above-described configuration ii). In the above-described configuration i), the occupancy ratio of portions to which the water repellency imparting agent is attached is 100%. If the occupancy ratio of portions to which the water repellency imparting agent is attached is excessively low, the aforementioned function of the non-skin-side water-repellent region 2 as the barrier layer is insufficient, and thus, there is a risk that the problems, such as a bodily fluid excreted from the body seeping into clothing and becoming externally visible, cannot be prevented. If the occupancy ratio of portions to which the water repellency imparting agent is attached is excessively high, the applied water repellency-imparting agent may penetrate even to the skin-facing surface 1a on the opposite side, depending on the type or the like of the underpants 1, when the water repellency-imparting agent is applied to the non-skin-facing surface 1b thereof so as to form the non-skin-side water-repellent region 2, and this may result in a decrease in the effect of preventing liquid seepage into the trousers 20. Moreover, if the occupancy ratio of portions to which the water repellency imparting agent is attached is high, the function of the non-skin-side water-repellent region 2 as the barrier layer is high, but on the other hand, there is a risk that the air permeability and the flexibility of the section where the non-skin-side water-repellent region 2 is formed will be insufficient.

In the water repellency-imparted underpants 10, the water repellency-imparting agent is not attached to the skin-facing surface 1a, which is located on the opposite side to the side on which the non-skin-side water-repellent region 2 is formed, or the water repellency-imparting agent is attached in an extremely small amount even if the agent is attached to the skin-facing surface 1a. Therefore, in the skin-facing surface 1a, the occupancy ratio of portions to which the water repellency imparting agent is attached is significantly lower than that of the non-skin-side water-repellent region 2. Specifically, in a 50-mm-square region (unit region) arbitrarily selected from the skin-facing surface 1a, the percentage of the total area of the portions to which the water repellency-imparting agent is attached (occupancy ratio of portions to which the water repellency imparting agent is attached) to the whole area of the 50-mm-square region is preferably 15% or less, more preferably 5% or less, and even more preferably zero.

To check whether or not a portion to which a water repellency-imparting agent is attached (a non-skin-side water-repellent region according to the present invention) is present in a fiber article, either one of the following checking methods A and B can be used. No matter which of the following checking methods A and B is used, the occupancy ratio of portions to which the water repellency imparting agent is attached can be calculated. However, in the case of the checking method B, in which a scanning electron microscope is used, the above-described unit region is set to be 2 mm square instead of 50 mm square, and in that case, the occupancy ratio of portions to which the water repellency imparting agent is attached is preferably 10% or greater, and more preferably 15% or greater, and is preferably 80% or less, and more preferably 60% or less.

Checking Method A: A colored liquid is dropped onto a surface (non-skin-facing surface) of a fiber article to be checked. The colored liquid is prepared by dissolving or dispersing an appropriate coloring agent (dye, pigment, or the like) in water. If no water repellency-imparting agent is attached to the position of the colored liquid dropped, the portion of the fiber article corresponding to the position of the colored liquid dropped is stained with that drop of the colored liquid. However, if a water repellency-imparting agent is attached to the position of the colored liquid dropped, the portion of the fiber article corresponding to the position of the colored liquid dropped is not stained and remains in the original color since the drop of the colored liquid is repelled by the water repellency-imparting agent. Usually, by performing the operation of dropping the colored liquid in this manner a plurality of times, a non-colored region that is not stained becomes visually observable, and the non-colored region can be determined to be a portion to which the water repellency-imparting agent is attached (non-skin-side water-repellent region in the present invention). The ratio of the area of the non-colored region to the area of a unit region (50 mm square), the former/the latter, is the above-described occupancy ratio of portions to which the water repellency imparting agent is attached.

If it is difficult to obtain the above-described ratio of the non-colored area in the checking method A to calculate the occupancy ratio of portions to which the water repellency imparting agent is attached, the following procedure can be performed. Specifically, about twenty drops of the colored liquid are substantially equally fallen onto a 50-mm-square unit region in a manner such that the drops do not overlap one another. The number of drops remaining (number of residual drops) on the unit region for 3 seconds or longer without being absorbed is counted, and the percentage of the number of residual drops to the total number of the fallen drops is taken as the occupancy ratio of portions to which the water repellency imparting agent is attached.

Checking Method B: This method is particularly effective in the case where the water repellency-imparting agent attached to an object to be checked is a modified silicone-containing water repellency-imparting agent. A surface of a fiber article to be checked (non-skin-facing surface) is observed with a scanning electron microscope (SEM) and an energy dispersive X-ray spectrometer (EDS) attached thereto, and an observation image is output if necessary, to check the presence or absence of the element Si contained in the modified silicone-containing water repellency-imparting agent. If the element Si is found, the portion where the element Si is found can be determined to be a portion to which the water repellency-imparting agent is attached (non-skin-side water-repellent region in the present invention). The magnification under an SEM is usually 50 to 100. For example, the portion to which the water repellency-imparting agent is attached shown in the image is copied to a transparent film, and the area percentage thereof is computed through image analysis, thereby obtaining the occupancy ratio of portions to which the water repellency imparting agent is attached. A specific example of the conditions for observation in the checking method B is as follows: A JSM-6510 from JEOL (manufactured by JEOL Ltd.) is used as the SEM, an EX-230BU (built-in) is used as the EDS, the magnification is 50, and the acceleration voltage is 10 kV.

In both of the checking methods A and B, there may be steps due to unevenness of the surface (non-skin-facing surface) of the fiber article to be checked, depending on the weave structure of the fabric of the fiber article, and in that case, the occupancy ratio of portions to which the water repellency imparting agent is attached is calculated with respect to the whole surface area of the unit region including the steps.

In the water repellency-imparted underpants 10, if the thickness 2T (see Fig. 2) of a portion to which the water repellency-imparting agent is attached, of the non-skin-side water-repellent region 2 is excessively small, there is a risk that the function of the non-skin-side water-repellent region 2 as the barrier layer will be insufficient. On the other hand, if the thickness 2T is excessively large, there is a risk that the air permeability and the flexibility will be pronouncedly reduced compared with the state before the water repellency-imparting agent is attached, that is, the underpants 1 (fiber article). Taking these into account, the thickness 2T of a portion to which the water repellency-imparting agent is attached, of the non-skin-side water-repellent region 2 is preferably 5% or greater, more preferably 10% or greater, and even more preferably 50% or greater, and, is preferably 60% or less, based on the thickness 1T (see Fig. 2) of the underpants 1 (fiber article). The ratio of the thickness 2T to the thickness 1T may hereinafter also be referred to as "thickness ratio of the water repellency-imparting agent". A particularly preferable range of the thickness ratio of the water repellency-imparting agent is from 50% to 60% as described above. Preferably, the underpants 1 are relatively thin while the thickness 2T is small, in view of maintaining as much as possible the properties of underwear intrinsic to the underpants 1, and effectively utilizing the water absorbency intrinsic to the underpants 1.

The thickness 2T of a portion to which the water repellency-imparting agent is attached, of the non-skin-side water-repellent region 2 is preferably 0.05 mm or greater and more preferably 0.1 mm or greater, and is preferably 0.5 mm or less.

The thickness 1T of the underpants 1 (fiber sheet 11) is preferably 0.5 mm or greater, and more preferably 0.8 mm or greater, and is preferably 2 mm or less, and more preferably 1.7 mm or less.

The thickness 2T can be measured using a known thickness measurement method, and the measurement may be facilitated by coloring the attached water repellency-imparting agent itself with an appropriate coloring agent prior to the measurement. When the attached water repellency-imparting agent is a modified silicone-containing water repellency-imparting agent, a measurement method that uses SEM-EDS as in the above-described checking method B is convenient. In that case, the thickness 2T is measured in the cross-sectional direction of the measurement sample. The observation may be performed under the conditions, for example, of a magnification of 100 and an acceleration voltage of 10 kV. When a measurement sample is prepared by cutting a piece having a predetermined shape from the water repellency-imparted underpants 10, a cutting method that is known to a person skilled in the art, such as use of a microtome, can be performed in order to prevent contamination of the cut surface to be observed due to the cutting operation, or alternatively, cutting may be performed with a FEATHER single edge blade razor (FAS-10) after freezing the water repellency-imparted underpants 10 with liquid nitrogen.

The above-described "ratio of the thickness 2T of a portion to which the water repellency-imparting agent is attached, of the non-skin-side water-repellent region to the thickness 1T of the fiber article" (thickness ratio of the water repellency-imparting agent) is measured in the following manner with the above-described SEM-EDS. Specifically, an object to be measured (fiber article to which a water repellency-imparting agent is attached) is frozen with liquid nitrogen, and then a portion of the object to be measured where a portion to which the water repellency-imparting agent is attached (non-skin-side water-repellent region) is present is cut in the thickness direction thereof with a FEATHER single edge blade razor (FAS-10). The cut surface is observed with SEM-EDS as in the checking method B, and the thickness 2T is measured. The thickness ratio of the water repellency-imparting agent is calculated from the found value and the thickness 1T. Preferably, the measurement of the thickness 2T is performed five times at different observation positions for each single sample, and an average value of the five found values of the thickness 2T is used to calculate the thickness ratio of the water repellency-imparting agent. The observation of the cut surface (measurement of the thickness 2T) can be performed, for example, with a JSM-6510 from JEOL (manufactured by JEOL Ltd.) as the SEM and an EX-230BU (built-in) as the EDS, at a magnification of 100 and an acceleration voltage of 10 kV.

In the water repellency-imparted underpants 10, the air permeance of a section where the non-skin-side water-repellent region 2 is formed is preferably 2 seconds/100 ml or greater, and more preferably 3 seconds/100 ml or greater, and is preferably 30 seconds/100 ml or less, and more preferably 20 seconds/100 ml or less. As used herein, the "section where the non-skin-side water-repellent region is formed" refers to a section including the non-skin-side water-repellent region 2 and a section of the water repellency-imparted underpants 10 (fiber sheet 11) that overlaps the non-skin-side water-repellent region 2 in a plan view, or in other words, refers to a section of the water repellency-imparted underpants 10 other than a section where the non-skin-side water-repellent region 2 is not formed.

If the air permeance of the section where the non-skin-side water-repellent region 2 is formed is excessively low, it can be determined that the occupancy ratio of portions to which the water repellency imparting agent is attached of the non-skin-side water-repellent region 2 is excessively high. This means that the air permeability and the flexibility are pronouncedly reduced, compared with those in a state before the attachment of the water repellency-imparting agent, that is, those of the underpants 1 (fiber article), and therefore, there is a risk that problems, such as dampness and rashes, with the skin of the wearer of the underpants will occur. If the air permeance of the section where the non-skin-side water-repellent region 2 is formed is excessively high (if there is substantially no difference between the air permeance of the section where the non-skin-side water-repellent region 2 is formed and the air permeance intrinsic to the underpants 1), it can be determined that the occupancy ratio of portions to which the water repellency imparting agent is attached of the non-skin-side water-repellent region 2 is excessively low. This means that the amount of water repellency-imparting agent attached is insufficient, and therefore, there is a risk that the function of the non-skin-side water-repellent region 2 as the barrier layer will be insufficient.

The air permeance is measured in accordance with JIS P8117 (1998), and is defined as the time required for 100 ml of air to pass through an area of 6.42 cm² under a certain pressure. Accordingly, high air permeance means that much time is required for air to pass through, that is, low air permeability. Conversely, low air permeance means high air permeability. In this manner, the magnitude of the air permeance and the level of the air permeability show an inverse relationship. The air permeance can be determined using an Oken air permeance tester.

Generally, if a fiber article is subjected to water-repellent treatment such as film coating, the air permeability intrinsic to the fiber article significantly decreases, inducing skin problems such as dampness and rashes. Taking this problem into account, it is preferable that the difference between the air permeance of a section where the non-skin-side water-repellent region 2 is formed and the air permeance of a section where the non-skin-side water-repellent region 2 is not formed (section in which the air permeance intrinsic to the underpants 1 is substantially maintained), i.e., the former - the latter, is 5 seconds/100 ml or less, provided that the former > the latter,.

In the water repellency-imparted underpants 10, the bending resistance of the section where the non-skin-side water-repellent region 2 is formed is preferably 60 mm or less, and more preferably 55 mm or less, and is preferably 10 mm or greater, and more preferably 15 mm or greater. The bending resistance of the section where the non-skin-side water-repellent region 2 is formed may also be an index of the occupancy ratio of portions to which the water repellency imparting agent is attached of the non-skin-side water-repellent region 2. Specifically, some of the water repellency-imparting agents (e.g., a modified silicone-containing water repellency-imparting agent, which will be described later) that are preferable as the water repellency-imparting agent for forming the non-skin-side water-repellent region 2 have the property of increasing the flexibility (reducing the stiffness) of a section to which the water repellency-imparting agent is attached, in accordance with an increase in the amount attached. When the non-skin-side water-repellent region 2 is formed of such a specific water repellency-imparting agent, it can be determined that the occupancy ratio of portions to which the water repellency imparting agent is attached of the non-skin-side water-repellent region 2 is high, if the bending resistance of the section where the non-skin-side water-repellent region 2 is formed is low, or specifically, if the bending resistance of the section where the non-skin-side water-repellent region 2 is formed is not substantially different from the bending resistance intrinsic to the underpants 1, or is lower than the intrinsic bending resistance. On the other hand, if the bending resistance of the section where the non-skin-side water-repellent region 2 is formed is significantly lower than the bending resistance intrinsic to the underpants 1, that is, if the section where the non-skin-side water-repellent region 2 is formed is excessively softened to an extent such that the stiffness (resilience) is significantly reduced, it can be determined that the occupancy ratio of portions to which the water repellency imparting agent is attached of the non-skin-side water-repellent region 2 is excessively high. In contrast to a modified silicone-containing water repellency-imparting agent, which will be described later, a common water-repellent coating agent has the property of reducing the flexibility of a section to which the coating agent is attached in proportion to the amount of coating agent attached. The reason for this is that a resin film is formed on the section to which the coating agent is attached. The bending resistance is measured by the following method.

### Method for Measuring Bending Resistance

The bending resistance is measured in accordance with the method A (45° cantilever method) specified in JIS L 1096 "8.21. measurement methods for bending resistance". However, for convenience of preparation (sampling) of measurement samples, only the bending resistance in the longitudinal direction of measurement samples is measured in the present measurement method. If a measurement sample has wrinkles, folds, and the like, the measurement sample is ironed before the measurement to eliminate the wrinkles, folds, and the like and then allowed to stand for twenty-four hours in the measurement environment for conditioning.

In view of preventing the presence of an excessive amount of the water repellency-imparting agent in the non-skin-side water-repellent region 2 to thereby provide the occupancy ratio of portions to which the water repellency imparting agent is attached thereof within an appropriate range, it is preferable that the bending resistance of the section where the non-skin-side water-repellent region 2 is formed be 60 mm or less as described above, and be also equal to or lower than the bending resistance of a section of the water repellency-imparted underpants 10 (underpants 1) where the non-skin-side water-repellent region 2 is not formed, that is, a water-repellent region non-formed portion. More specifically, the difference between the bending resistance of the water-repellent region non-formed portion and the bending resistance of the section where the non-skin-side water-repellent region 2 is formed, i.e., the former - the latter, is preferably 0 to 25 mm, and more preferably 0 to 20 mm, provided that the former > the latter. For example, an example of the water repellency-imparting agent that can establish this relationship in magnitude "(bending resistance of water-repellent region non-formed portion) > (bending resistance of section where non-skin-side water-repellent region 2 is formed)" is a composition containing a modified silicone (modified silicone-containing water repellency-imparting agent), which will be described later. When this composition is used as the water repellency-imparting agent for forming the non-skin-side water-repellent region 2, it is preferable that the difference between the former and the latter (the former - the latter) be within the above-described specific range. Examples of the modified silicone contained in the composition include polyoxazoline modified silicones and polyether modified silicones with various molecular weights and branching structures, and these modified silicones can be used singly or in a combination of two or more.

In the water repellency-imparted fiber article of the present invention, it is necessary that the section to which the water repellency-imparting agent is attached, of the fiber article serving as the base thereof include the non-skin-facing surface of the fiber article, which is the section where the non-skin-side water-repellent region is formed. However, with regard to the "section of the fiber article that overlaps the non-skin-side water-repellent region in a plan view", or specifically, for example, the section of the underpants 1 (fiber sheet 11) that overlaps the non-skin-side water-repellent region 2 in a plan view in the case of the water repellency-imparted underpants 10 shown in Fig. 2, the water repellency-imparting agent may or may not be attached to that section, provided that the water absorbing layer in which the above-described water absorbency, that is, the "water absorbency in terms of a water absorption time of 30 seconds or less as measured in accordance with the dropping method of JIS L-1907" is maintained is present in that section. Even when the fiber article has a section to which the water repellency-imparting agent is attached, the above-described water absorbency may be maintained in that section depending on the amount of water repellency-imparting agent attached, the attaching pattern of the water repellency-imparting agent, and the like.

With respect to the attachment of the water repellency-imparting agent, the amount of water repellency-imparting agent attached may decrease from the non-skin-facing surface 1b of the underpants 1 (fiber article) toward the skin-facing surface 1a thereof, in the water repellency-imparted underpants 10 (water repellency-imparted fiber article) shown in Fig. 2 as an embodiment of the water repellency-imparted fiber article of the present invention. In this case where the amount of water repellency-imparting agent attached decreases, no water repellency-imparting agent may be attached to the skin-facing surface 1a side (region spanning from the skin-facing surface 1a for a predetermined length in the thickness direction of the underpants 1). As will be described later, the non-skin-side water-repellent region 2 is typically formed by applying the water repellency-imparting agent to the non-skin-facing surface 1b of the underpants 1 with a spray or the like. When the water repellency-imparting agent is applied from the non-skin-facing surface 1b side of the underpants 1 in this manner, the applied water repellency-imparting agent is attached to only the non-skin-facing surface 1b to form the non-skin-side water-repellent region 2 thereon, or the applied water repellency-imparting agent forms the non-skin-side water-repellent region 2 and further penetrates into the underpants 1 (fiber sheet 11). In both cases, the above-described "state in which the amount of water repellency-imparting agent attached decreases from the non-skin-facing surface of the fiber article toward the skin-facing surface thereof' is consequently obtained. As described above, Fig. 4(a) shows a non-skin-facing surface of a specific example of this state, and Fig. 4(b) shows a skin-facing surface on the opposite side to the non-skin-facing surface.

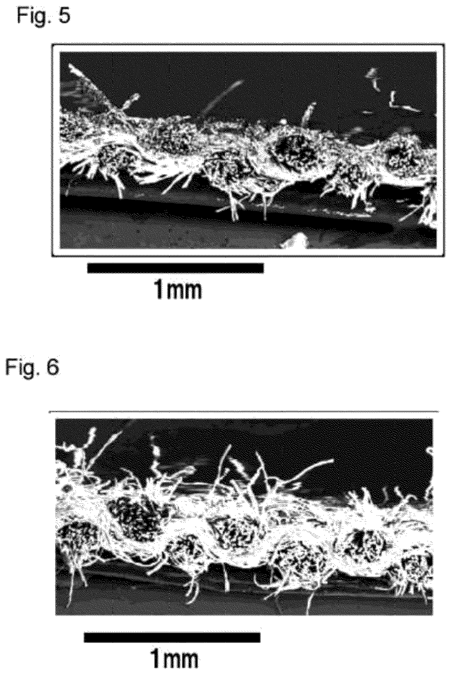

Fig. 5 shows an EDS image (at a magnification of 100) of a cross section taken along the thickness direction of a specific example of the above-described "state in which the amount of water repellency-imparting agent attached decreases from the non-skin-facing surface of the fiber article toward the skin-facing surface". In Fig. 5, a white portion extending in the lateral direction of the image at the middle of the image in the vertical direction thereof is the fiber article (constituent fibers), and minute dots (black spots) that are present in the substantially upper half of the white portion are the water repellency-imparting agent, or more specifically, the element Si contained in a modified silicone-containing water repellency-imparting agent, which is a type of water repellency-imparting agent. In the specific example of the water repellency-imparted fiber article of the present invention shown in Fig. 5, the above-described dots are present such that a relatively large number of dots are located on the non-skin-facing surface side (upper surface side of the sheet in Fig. 5), and that a relatively small number of dots are located on the skin-facing surface side (lower surface side of the sheet in Fig. 5). When the amount of water repellency-imparting agent attached decreases from the non-skin-facing surface 1b toward the skin-facing surface 1a in this manner, it is unlikely that the skin-facing surface 1a side (skin-facing surface 1a and the vicinity thereof) of the water repellency-imparted underpants 10 is affected by the water repellency-imparting agent, and therefore, the tactile feel intrinsic to the underpants 1 is retained, which is even more preferable.

Moreover, with respect to the attachment of the water repellency-imparting agent, the water repellency-imparted underpants 10 as shown in Fig. 2 as an embodiment of the water repellency-imparted fiber article of the present invention may have a skin-side water-repellent region (not shown) on the skin-facing surface 1a of the underpants 1 (fiber article), the skin-side water-repellent region including a portion to which the water repellency-imparting agent is attached. In this case where the skin-side water-repellent region is formed, the amount of water repellency-imparting agent attached per unit area in the skin-side water-repellent region is smaller than that in the non-skin-side water-repellent region 2. Moreover, in this case where the skin-side water-repellent region is formed, the water absorbing layer in which the above-described water absorbency, that is, the "water absorbency in terms of a water absorption time of 30 seconds or less as measured in accordance with the dropping method of JIS L-1907" is maintained is present between the non-skin-side water-repellent region 2 and the skin-side water-repellent region. In the case where the skin-side water-repellent region is formed, the water repellency is imparted to the skin-facing surface of the fiber article by forming the skin-side water-repellent region thereon, and therefore, the effect of reducing the sticky feel to the skin of the user can be obtained. The ratio of the amount of water repellency-imparting agent attached in the skin-side water-repellent region to the amount of water repellency-imparting agent attached in the non-skin-side water-repellent region, the former/the latter, is preferably 1/5 or less, and more preferably 1/10 or less, and it is also preferable that no water repellency-imparting agent is observed in the skin-side water-repellent region.

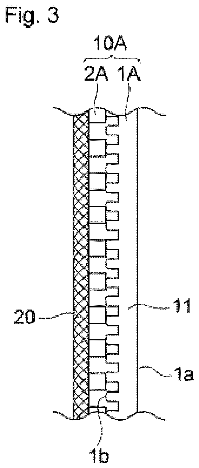

Fig. 3 shows another embodiment of the water repellency-imparted fiber article of the present invention. For the other embodiment described below, components that are different from those of the above-described water repellency-imparted underpants 10 will be described primarily, and the same components are denoted by the same reference numerals as in the above-described embodiment to omit their descriptions. Descriptions of the water repellency-imparted underpants 10 are appropriately applied to those components that will not be particularly described.

In a pair of water repellency-imparted underpants 10A (water repellency-imparted fiber article), which are a piece of inner clothing, shown in Fig. 3, the skin-facing surface 1a of a pair of underpants 1A (fiber article) is a flat surface with substantially no unevenness, whereas the non-skin-facing surface 1b thereof has an uneven structure constituted by a plurality of projections and depressions. A water repellency-imparting agent that forms a non-skin-side water-repellent region 2A is attached to the projections constituting the uneven structure. The water repellency-imparting agent is not attached to the depressions constituting the uneven structure, or even if the water repellency-imparting agent is attached to the depressions, the amount attached thereto is smaller than the amount attached to the projections. The uneven structure of the non-skin-facing surface 1b of the underpants 1A can be formed by a known embossing process, such as an embossing process with or without heat, an ultrasonic embossing process, or the like. When the material of the fiber sheet 11 of the underpants 1A is a woven fabric or a knitted fabric, the uneven structure can also be given by devising the weaving or knitting pattern appropriately.

The "uneven structure" herein means a structure constituted by a plurality of projections that are formed by the weave or knit texture and depressions that are formed between the plurality of projections. When 60% or greater of the plurality of depressions that are present on the surface (non-skin-facing surface) of the fiber article have a larger area than a square of side 500 µm in a plan view and also have a depth (length from the top of a projection adjacent to the depression to the bottom of that depression) of 500 µm or greater, it can be said that the surface (non-skin-facing surface) of the fiber article has an uneven structure.

For example, with respect to the non-skin-side water-repellent region 2 shown in Fig. 2, the skin-facing surface 1a of the underpants 1, on which the non-skin-side water-repellent region 2 is formed, is a flat surface with substantially no unevenness, and therefore, the surface of the non-skin-side water-repellent region 2 also is a flat surface with substantially no unevenness. In this case, almost the entire area of the non-skin-side water-repellent region 2 can come into contact with the trousers 20, which are a piece of outer clothing. Thus, vapor that is produced in an inner space (between the skin of the wearer of the underpants and the skin-facing surface 1a of the underpants 1) of the underpants 1 when worn is unlikely to be discharged to the outside. Therefore, there is concern that the trousers 20 will get wet with the vapor, and there also is concern that skin problems such as dampness and rashes will occur. This vapor is produced by evaporation of bodily fluids, such as urine and sweat, that are excreted by the wearer of the underpants due to body heat. In contrast, with respect to the non-skin-side water-repellent region 2A shown in Fig. 3, the surface of the non-skin-side water-repellent region 2A that faces the trousers 20 has the uneven structure. Accordingly, the area thereof contact with the trousers 20 is reduced compared with that of the non-skin-side water-repellent region 2, and a gap that can function as an air passage may be formed in a portion that is in contact with the trousers 20. Therefore, the above-described concern is eradicated. In this manner, the water repellency-imparted underpants 10A, which includes the non-skin-side water-repellent region 2A having the uneven surface, has an excellent quick-drying, so that the sticky feel is even further reduced.

Hereinafter, the water repellency-imparting agent used in the present invention will be described.

The water repellency-imparting agent that is used in the present invention is required to be able to impart water repellency (waterproofness) to the fiber article and form a non-skin-side water-repellent region that has a water contact angle and a water absorption rate within the above-described specific ranges.

Moreover, as described above, in the water repellency-imparted fiber article of the present invention, the "water absorbing layer" maintaining the water absorbency intrinsic to the fiber article needs to be present in a section of the fiber article that overlaps the non-skin-side water-repellent region in a plan view and on a side that is closer to the skin of the user than the non-skin-side water-repellent region. When the water repellency-imparting agent is applied to one side (non-skin-facing surface) of the fiber article, it is difficult to secure the above-described water absorbing layer if the applied water repellency-imparting agent highly penetrate to transfer to the other side (skin-facing surface), and furthermore, in this case, there is concern that the air permeability, the flexibility, and the like of the fiber article will be significantly reduced.

The water repellency-imparting agent is therefore required not only to be able to impart water repellency to the fiber article but also to have low penetrability in the fiber article and, when applied to the fiber article, stay at the applied position and the vicinity thereof (non-penetrability).

An example of the water repellency-imparting agent that is used in the present invention is a composition containing a modified silicone (modified silicone-containing water repellency-imparting agent). The modified silicone is a component that functions as a water repellent and imparts water repellency to the fiber article. Examples of the modified silicone include polyoxazoline modified silicones and polyether modified silicones with various molecular weights and branching structures, and these modified silicones can be used singly or in a combination of two or more. Among these modified silicones, a polyoxazoline modified silicone containing 55% to 98% of a dimethyl siloxane structure, such as a silicone unit, can achieve both the stability of water repellency after treatment and the ability to be washed off through a common washing operation, and is therefore preferably used in the present invention. In view of obtaining stable water repellency, the modified silicone content in the water repellency-imparting agent is preferably 25 mass% or greater, and more preferably 30 mass% or greater with respect to the total mass of the water repellency-imparting agent. When a composition containing a modified silicone is used as the water repellency-imparting agent, the modified silicone, which is a component of the composition and serves as the main causative substance of water repellency, deposits, in preference to the other components of the composition, on the surface (surface of the non-skin-side water-repellent region) of the portion of the fiber article to which the composition is attached, and therefore, an advantage that the composition can be used (attached) in a relatively small amount can be obtained.

The composition containing a modified silicone may also contain a volatile solvent in which the modified silicone is soluble. In other words, the water repellency-imparting agent that is used in the present invention may be in a liquid form in which the modified silicone is dissolved in the volatile solvent at normal temperature and pressure. The non-skin-side water-repellent region is formed by applying the water repellency-imparting agent in this liquid form to the non-skin-facing surface of the fiber article, and the water repellency-imparted fiber article of the present invention is thus obtained. In the thus obtained water repellency-imparted fiber article, the volatile solvent contained in the water repellency-imparting agent in this liquid form may have volatilized and no longer remain. Examples of the volatile solvent include ethanol, methanol, isopropyl alcohol, and water/ethanol mixed liquids with various mixing ratios, and these volatile solvents can be used singly or in a combination of two or more. Among these volatile solvents, ethanol has high safety and volatility (quick-drying ability), and is therefore preferably used in the present invention.

The composition containing a modified silicone may also contain a water-soluble binder. The water-soluble binder primarily functions to fix the modified silicone, which is a water repellent, to an object (fiber article) to which the composition is applied. Binders of this type include non-water-soluble binders that are insoluble or semi-soluble in water, and water repellency-imparting agents in commercially-available waterproofing sprays for clothes often contain a non-water-soluble binder. However, if a water repellency-imparting agent containing a non-water-soluble binder is applied to a fiber article, yellowing and stiffening of the fiber article, an unpleasant solvent odor, and the like tend to occur, and also there may be limitations on washing of the fiber article. Thus, the handleability of the fiber article may be reduced. In contrast, when a water-soluble binder is selected, these problems caused by use of a non-water-soluble binder are unlikely to occur.

Examples of the water-soluble binder include polyvinyl alcohols, acrylic resins, and acrylic resin emulsions, and these water-soluble binders can be used singly or in a combination of two or more. Among these water-soluble binders, various acrylic resins, such as (vinyl methyl ether/butyl maleate) copolymers typified by "Gantrez ES-425" available from Matsumoto Trading Co., Ltd., and acrylic resin alkanolamines are strongly attached to the surface of the fiber article via an ester bond, and are therefore preferably used in the present invention. Such strong attachment of various acrylic resins to the fiber article via an ester bond is especially pronounced in the case where such an acrylic resin is incorporated into a polyether modified silicone-containing water repellency-imparting agent and, furthermore pronounced by a heating operation, such as ironing or drying, in such a case. The water-soluble binder content in the water repellency-imparting agent is preferably 20 mass% or greater, and more preferably 30 mass% or greater, and is preferably 80 mass% or less, and more preferably 70 mass% or less, with respect to the total mass of the water repellency-imparting agent.

In an embodiment of the water repellency-imparted fiber article of the present invention, the water repellency (waterproofness) can be removed by washing the water repellency-imparted fiber article with water by a usual method. That is to say, in an embodiment of the water repellency-imparted fiber article of the present invention, washing of the water repellency-imparted fiber article can removes the water repellency-imparting agent to eliminate the non-skin-side water-repellent region, thereby turning the water repellency-imparted fiber article into the original fiber article. Such a water repellency-imparted fiber article whose water repellency can be removed through washing is especially useful in the case where the water-repellent treatment of a fiber article with a water repellency-imparting agent (production of a water repellency-imparted fiber article) is performed privately or in the home, rather than industrially. In other words, for example, a series of operations of "washing the water repellency-imparted fiber article after use to turn the water repellency-imparted fiber article into the original fiber article; then storing the fiber article; and performing the water-repellent treatment of the fiber article with the water repellency-imparting agent if the need to impart water repellency to the fiber article arises thereafter" can be repeatedly performed privately or in the home.

The above-described "water repellency-imparted fiber article whose water repellency can be removed by washing" can specifically be defined as follows. A water repellency-imparted fiber article is immersed in a liquid containing 0.1 mass% of a commonly available detergent for clothing and ion exchanged water under stirring for 10 minutes, and then the water repellency-imparted fiber article is lightly squeezed and further immersed in ion exchanged water under stirring at a rotational speed of 350 rpm for 10 minutes. If the amount of water repellency-imparting agent contained in the water repellency-imparted fiber article after such a washing procedure is 20 mass% or less based on that before the washing procedure, the water repellency-imparted fiber article is regarded as being a water repellency-imparted fiber article whose water repellency can be removed by washing. A surfactant including mainly of a long-chain alkyl ether sulfate or the like can be used as the "commonly available detergent for clothing", and common commercially available detergents for clothing can generally be used. Specifically, for example, a liquid detergent for clothing "Attack Neo" (registered trademark) manufactured by Kao Corporation can be used. The state where "the water repellency-imparted fiber article is lightly squeezed" can be specifically exemplified by the following: twenty sheets of filter paper "2" of a size larger than a fiber article to be squeezed are laid one on top of another, the fiber article is placed on top of the stack of the filter paper sheets, and a pressure is applied to the whole by passing a 5-kg roller (width: 50 mm) back and forth to thereby squeeze the fiber article.

Supplementary explanations of the conditions and the like of the washing of the water repellency-imparted fiber article will be given below. Conditions, such as the volume of a tub that is used for immersion of the water repellency-imparted fiber article under stirring, may be set as appropriate within the range of conditions disclosed herein. Favorable conditions for washing a single fiber article may be, for example, as follows: a beaker having a volume of 2 L is used as the tub for immersion of the water repellency-imparted fiber article under stirring, and 1.5 L of washing liquid and 1.5 L of rinsing liquid are used. Moreover, for stirring, a stirrer whose rotational speed can be monitored can be used as appropriate, and preferably, a magnetic stirrer HS-50D manufactured by AS ONE Corporation is used in a combination with an AS ONE Crosshead Rotor Double (diameter: 60 mm, product number: 1-5409-07) as a stirrer bar.

Fig. 6 shows a specific example of the water repellency-imparted fiber article of the present invention after immersion under stirring (i.e., after washing) in accordance with the above-described procedures. Before washing, this water repellency-imparted fiber article is the water repellency-imparted fiber article shown in Fig. 5, in which the amount of water repellency-imparting agent attached decreases from the non-skin-facing surface of the fiber article toward the skin-facing surface. Fig. 6 is an EDS image (at a magnification of 100) of a cross section of that specific example taken along the thickness direction thereof. In the water repellency-imparted fiber article before washing in Fig. 5, the water repellency-imparting agent (element Si derived from the modified silicone-containing water repellency-imparting agent) can be observed as a large number of dots (black spots) on the non-skin-facing surface side (upper surface, and the vicinity thereof, of the white portion in Fig. 5) thereof; however, it can be seen that the water repellency-imparting agent have almost completely disappeared in the water repellency-imparted fiber article after washing shown in Fig. 6. For this reason, it can be understood that the specific example of the water repellency-imparted fiber article of the present invention shown in Fig. 5 is the "water repellency-imparted fiber article whose water repellency can be removed by washing".

The percentage of the water repellency-imparting agent remaining in the water repellency-imparted fiber article after washing can be actually measured through sampling, or alternatively it can be roughly measured by placing a drop of liquid on the water repellency-imparted fiber article and observing the state of the liquid absorption, for convenience's sake. Specifically, for example, the percentage of the water repellency-imparting agent remaining on that surface can be determined as zero in the following case: with respect to the water-repellency of the non-skin-facing surface (non-skin-side water-repellent region) of the water repellency-imparted fiber article, the non-skin-facing surface of the water repellency-imparted fiber article before washing exhibits relatively strong water repellency, specifically, a water contact angle of 80 degrees or greater as measured by the above-described method, and after washing, that non-skin-facing surface exhibits a contact angle equal to that of the skin-facing surface of the water repellency-imparted fiber article, or absorbs so instantly the drop of ion exchanged water used in the measurement of the contact angle that it is impossible to measure the contact angle.

The "water repellency-imparted fiber article whose water repellency can be removed through washing" can be obtained by using the above-described water-soluble binder as the binder contained in the water repellency-imparting agent. The "water repellency-imparted fiber article whose water repellency can be removed by washing" can also be obtained by using, as the water repellency-imparting agent, a modified silicone-containing water repellency-imparting agent that contains a modified silicone having a hydrophilic polymer as a hydrophilic side chain as a modified group. In the latter case, the whole of the water repellency-imparting agent may be composed of one or more of such modified silicone-containing water repellency-imparting agents, or the water repellency-imparting agent may be composed mainly of one or more of such modified silicone-containing water repellency-imparting agents and further contain one or more other water repellency-imparting agents in a smaller amount. In such a modified silicone-containing water repellency-imparting agent, the mass ratio of the hydrophilic side-chain content to the silicone principal-chain content is preferably from 55 to 98 mass%.

The water repellency-imparted fiber article of the present invention may also contain, as a measure against odor (also referred to as an odor-preventing agent), if necessary, at least one selected from the group consisting of a deodorizing ingredient, an odor-removing ingredient, a fragrant ingredient, an antimicrobial agent, a bactericidal agent, and a refreshing agent. The odor-preventing agent may be contained in the fiber article or the non-skin-side water-repellent region constituting the water repellency-imparted fiber article. For example, in a water repellency-imparted fiber article obtained by applying a water repellency-imparting agent (composition containing a modified silicone) containing an odor-preventing agent to a fiber article that does not contain an odor-preventing agent, the odor-preventing agent is contained in at least the non-skin-side water-repellent region, and depending on the conditions for applying the water repellency-imparting agent and other conditions, the odor-preventing agent may also be contained in the fiber article. These measures against odor are also preferable in light of the effect of suppressing rashes and the like of the skin. Preferably, which of the above-described ingredients and agents is used as the measure against odor is determined with consideration given to the influence on, for example, the safety of the body of the user at a section for which that ingredient or agent is used.

Examples of the deodorizing ingredient and the odor-removing ingredient include known odor removers, such as particles having a layered structure and made of activated carbon, silica gel, zeolite, or a zirconium phosphate; particles having a stereostructure made of a silicate; and zinc oxide, and these odor removers can be used singly or in a combination of two or more.

A natural fragrance or a synthetic fragrance may be used in an appropriate amount as the fragrant ingredient. Examples of the natural fragrance include fragrances extracted from naturally occurring substances, such as green tea, benzoin, clove oil, jasmine absolute, mate, mimosa, Tonkin musk, frankincense, rosemary oil, sandalwood oil, vetiver oil, and violet leaf absolute, and examples of the synthetic fragrance include higher alcohols, aldehydes, benzaldehydes, benzoic acid, cinnamic acid, cinnamaldehydes, cinnamyl alcohols, coumarin, esters, indole, ketones, alicyclic acid and related compounds, terpenoids, and vanillin. These fragrances can be used singly or in a combination of two or more.

As the antimicrobial agent and the bactericidal agent, any of known antimicrobial and bactericidal substances can be used. Examples thereof include silver, copper, zinc, silica, activated carbon, aluminosilicate compounds, zeolite, electrolytes that can be used as moisture absorbing materials, and alcohols, aldehydes, phenols, hydrogen peroxides, chlorine, hypochlorites, and surfactants. These substances can be used singly or in a combination of two or more.

A refreshing agent disclosed in Japanese Patent No. 6121269, for example, can be appropriately used as the refreshing agent. When a refreshing agent is contained the water repellency-imparted fiber article, the refreshing agent imparts a feel of refreshment and a dry feel to the water repellency-imparted fiber article, and thus, the comfort can be further improved.

As described above, the water repellency-imparted fiber article of the present invention has water absorbency, which is intrinsic to the fiber article, and water repellency, which is provided by the non-skin-side water-repellent region, and can be applied to various uses by making the most of these features. For example, the water repellency-imparted fiber article can be applied to inner clothing typified by underwear, such as underpants, loincloths, shirts, brassieres, girdles, and socks; sporting clothing, such as football shirts, golf shirts, tennis shirts, basketball shirts, table tennis shirts, badminton shirts, running shirts, football pants, tennis pants, basketball pants, table tennis pants, badminton pants, running pants, golf pants, various types of sporting undershirts, various types of sporting innerwear, sweaters, T-shirts, jerseys, sweat shirts, windbreakers, shorts, leggings, and the like; products for light incontinence, such as incontinence pads, sanitary napkins, sheets designed to absorb vaginal discharge, such as party-liners, breastfeeding pads, and the like. The location of the non-skin-side water-repellent region in the water repellency-imparted fiber article of the present invention can be appropriately set according to the use of the water repellency-imparted fiber article. The location of the non-skin-side water-repellent region is set, at least on the non-skin-facing surface of the fiber article, at a section where liquid seepage is expected to occur in the intended use, for example, a section corresponding to an excretion part, such as the penis, an armpit, the back, a crotch portion, the buttocks, or a nipple portion.

An incontinence pad typically includes a topsheet that is disposed at a position relatively close to the skin of a wearer, a backsheet that is disposed at a position relatively away from the skin of the wearer, and an absorbent member that is interposed between the topsheet and the backsheet. When the incontinence pad is worn, the backsheet may come into contact with outer clothing such as a pair of trousers. Accordingly, in the case where the water repellency-imparted fiber article of the present invention is applied to an incontinence pad, the water repellency-imparting agent can be applied to an outer surface (non-skin-facing surface) of the backsheet of the pad to form a non-skin-side water-repellent region thereon.

A method for producing the water repellency-imparted fiber article of the present invention includes the step of applying a water repellency-imparting agent to a non-skin-facing surface of a fiber article having the above-described water absorbency, that is, the "water absorbency in terms of a water absorption time of 30 seconds or less as measured in accordance with the dropping method of JIS L-1907". The water repellency-imparted fiber article of the present invention can basically be produced simply by performing this step, and therefore can be efficiently produced without the necessity for a large-sized equipment or a complicated procedure. Therefore, the method for producing the water repellency-imparted fiber article of the present invention can be performed not only industrially but also privately or in the home.

In the method for producing the water repellency-imparted fiber article of the present invention, the water repellency-imparting agent need not be applied to the skin-facing surface of the fiber article, or the water repellency-imparting agent may be applied thereto in a smaller amount than that applied to the non-skin-facing surface of the fiber article, as described above.

There is no particular limitation on the method for applying the water repellency-imparting agent to the fiber article. The application means may be of a contact type that comes into contact with the fiber article when applying the agent thereto, or of a contactless type that does not come into contact with the fiber article when applying the agent thereto. As described above, in light of the air permeability, the flexibility, and other properties, the attaching pattern of the water repellency-imparting agent in the non-skin-side water-repellent region is preferably a mixed pattern in which both a portion to which the water repellency-imparting agent is attached and a portion to which the water repellency-imparting agent is not attached intermingle, rather than a pattern in which the water repellency-imparting agent is continuously present over the entire area of the non-skin-side water-repellent region while leaving no gap (such as daubing with the water repellency-imparting agent). In view of reliably obtaining such a mixed pattern, it is preferable that, in producing the water repellency-imparted fiber article of the present invention, the application of the water repellency-imparting agent to the fiber article is performed by spraying the water repellency-imparting agent with a contactless application means, such as a spray.

Moreover, in the method for producing the water repellency-imparted fiber article of the present invention, the fiber article may be dried after the water repellency-imparting agent is applied to the fiber article. To dry the water repellency-imparting agent, drying may be performed by heat, vacuum, or combination thereof (forced drying), or air drying may be performed. In the case of drying by heat, it is preferable that drying be performed without damaging a base material of the fiber article and the water repellency-imparting agent. In particular, when the water repellency-imparting agent that is applied to the fiber article is the above-described liquid material containing a volatile solvent and a modified silicone dissolved therein, the volatile solvent can be reliably removed by introducing the drying step. In that case, the conditions for drying the fiber article after the water repellency-imparting agent is applied thereto can be appropriately set according to the components, including the volatile solvent, of the water repellency-imparting agent; however, in view of allowing the water repellency-imparting agent applied and attached to the fiber article to fully perform its function to thereby achieve both the air permeability and the water repellency, as well as in view of preventing dimensional shrinkage and impregnation with the water repellency-imparting agent, the fiber article is more preferably dried under a condition that the temperature of the applied water repellency-imparting agent is 50°C or less.

Examples of the method for applying and attaching the water repellency-imparting agent to the fiber article include immersion in a solution, spray coating, dipping, and a roll-on method as well as coating using a known liquid coating apparatus, including coating that follows a printing method such as transfer printing, die coating, gravure coating, inkjet printing, and screen printing. These methods can be arbitrarily used. Moreover, in the case where the water repellency-imparting agent is solidified, the following method can be adopted. Specifically, for example, the water repellency-imparting agent solidified into a stick shape is brought into direct contact with the fiber article to thereby apply the water repellency-imparting agent thereto, or the water repellency-imparting agent is indirectly applied to the fiber article using a sponge or the like. Of these methods, spray coating is preferable because a desired state can be efficiently provided by appropriately adjusting the properties, such as viscosity, of the water repellency-imparting agent, the shape of a spray nozzle, the amount of water repellency-imparting agent applied, and other conditions. In particular, a method in which an aerosol sprayer, a manual trigger sprayer, ultrasonic waves, or the like is used for applying and attaching the water repellency-imparting agent is more preferable because the user can form a water-repellent layer in a simple and easy manner. Moreover, when applying the water repellency-imparting agent, it is necessary to appropriately adjust the conditions in order to control the penetrability of the agent, including the viscosity of the blended composition as the water repellency-imparting agent, the pressure applied to the fiber article such as a fabric cloth, the moving speed of the sprayer, and the textile printing speed.

An example of the application means that can be used in the production method of the present invention is a pump spray-type application means in which the water repellency-imparting agent (composition containing a modified silicone) is put into a container equipped with a sprayer. That is to say, in the method for producing the water repellency-imparted fiber article of the present invention, the application of the water repellency-imparting agent to the fiber article can be performed by spraying the water repellency-imparting agent using a pump spray-type application means. In the present invention, conventionally known pump spray-type application means can be used without limitation.

Another example of the application means that can be used in the production method of the present invention is an aerosol spray-type application means in which the water repellency-imparting agent (composition containing a modified silicone) and a propellant are put into a pressure-resistant container for aerosol spray. That is to say, in the method for producing the water repellency-imparted fiber article of the present invention, the application of the water repellency-imparting agent to the fiber article can be performed by spraying the water repellency-imparting agent using an aerosol spray-type application means in which the water repellency-imparting agent and a propellant are put into a pressure-resistant container for aerosol spray. In the present invention, conventionally known aerosol spray-type application means can be used without limitation. Examples of the propellant include those that use a compressed gas, such as a nitrogen gas or a carbonic acid gas, and those that use a liquefied gas, such as a liquefied petroleum gas (LPG) or dimethyl ether (DME).

Still another example of the application means that can be used in the production method of the present invention is a manual spray-type application means in which the water repellency-imparting agent (composition containing a modified silicone) is put into a container equipped with a manual sprayer. That is to say, in the method for producing the water repellency-imparted fiber article of the present invention, the application of the water repellency-imparting agent to the fiber article can be performed by spraying the water repellency-imparting agent using a manual spray-type application means in which the water repellency-imparting agent is put into a container equipped with a manual sprayer. Such a manual spray-type application means is a sprayer that does not use any propellant such as a gas, and specific examples thereof include a manual trigger sprayer and an ultrasonic sprayer. In particular, a manual spray-type application means with an accumulator provides favorable small mist particle size, uniformity of mist particle size, and the like, and is suitably used in the present invention. Moreover, as for one example of the method for using the manual spray-type application means, the water repellency-imparting agent can be sprayed with compressed air or the like using a container equipped with a compressor.

Moreover, yet another example of the application means that can be used in the production method of the present invention is an application means including a stick-shaped solidified matter of the water repellency-imparting agent (composition containing a modified silicone), the application means being configured such that the water repellency-imparting agent can be applied to an object by bringing the solidified matter into contact with that object. That is to say, in the method for producing the water repellency-imparted fiber article of the present invention, the application of the water repellency-imparting agent to the fiber article can be performed by using an application means that includes a stick-shaped solidified matter of the water repellency-imparting agent and bringing the solidified matter into contact with an object. Such an application means including the stick-shaped solidified matter can be configured in the same manner as, for example, lipstick, a glue stick, and the like, and includes the stick-shaped solidified matter of the water repellency-imparting agent and a support portion that supports the solidified matter.

Moreover, yet another example of the agent application means that can be used in the production method of the present invention is a roll-on application means in which the water repellency-imparting agent (composition containing a modified silicone) is put into a roll-on container. That is to say, in the method for producing the water repellency-imparted fiber article of the present invention, the application of the water repellency-imparting agent to the fiber article can be performed using a roll-on application means in which the water repellency-imparting agent is put into a roll-on container. The roll-on container has, in the opening thereof, an inner stopper in which a ball is freely rotatably held, and is configured to distribute the content (water repellency-imparting agent) over a ball surface and apply the content to a desired section. To use the roll-on container, while the ball is in contact with the non-skin-facing surface of the fiber article as an object to which the content is to be applied, the main body of the container is raised up to cause the content to come into contact with the ball, and the ball is rolled on the non-skin-facing surface to thereby distribute the content over the ball surface. Known roll-on containers can be used as the roll-on container without limitation.

Information about the usage method of the application means may be provided, so that the information is externally visible, on the application means that is used to apply the water repellency-imparting agent to the fiber article in the production method of the present invention, including the above-described pump spray-type application means, aerosol spray-type application means, manual spray-type application means, application means including a stick-shaped solidified matter, or roll-on agent application means. In particular, in the case where the treatment method according to the present invention is performed privately or in the home, the user can be effectively prevented from incorrectly using the application means and therefore can smoothly perform the operation for imparting water-repellency to fiber article, if information about the application means for use in the method is provided externally visibly.

The above-described preferred embodiments can be implemented by appropriately selecting the formulation of the water repellency-imparting agent, the application method, and other conditions. For example, in the case where the water repellency-imparting agent is a spray formulation, in order to selectively attach the water repellency-imparting agent to the non-skin-facing surface of the fiber article while maintaining water absorbency of the skin-facing surface, which is located on the opposite side, the following conditions are preferably combined: the ratio of the volatile solvent to the water repellency-imparting agent and the binder is 80 mass% or less; dimethyl ether, in which a modified silicone can dissolve, is used as the spray propellant (gas agent) in an amount of 50 mass% or greater; and, furthermore, the amount of spray propellant is set to be one to three times larger than the amount of the main agent composed of the water repellency-imparting agent, the binder, and the solvent. When these conditions are used, the water repellency-imparting agent with high viscosity and quick-drying ability can be attached to the surface (non-skin-facing surface) of the fiber article. It is also preferable to warn the user to keep the spray at a distance of at least 15 cm from the object and complete spraying in at most three to-and-fro motions of spraying.

Although the present invention has been described above based on embodiments thereof, the present invention is not limited to the foregoing embodiments, and changes and modifications can be made thereon as appropriate. In relation to the above-described embodiments of the present invention, the following additional remarks will be further disclosed.

1. A water repellency-imparted fiber article, comprising:
   a fiber article having a skin-facing surface to be disposed on a side that is relatively close to the skin of a user during use and a non-skin-facing surface to be disposed on a side that is relatively away from the skin of the user, the fiber article containing a water-absorbent fiber and having water absorbency in terms of a water absorption time of 30 seconds or less as measured in accordance with the dropping method of JIS L-1907; and
   a water repellency-imparting agent attached to the fiber article,
   wherein the water repellency-imparted fiber article has a non-skin-side water-repellent region on the non-skin-facing surface of the fiber article, the non-skin-side water-repellent region including a portion to which the water repellency-imparting agent is attached,
   the water repellency-imparted fiber article has a water absorbing layer in a section of the fiber article that overlaps the non-skin-side water-repellent region in a plan view and on a side that is closer to the skin of the user than the non-skin-side water-repellent region, the water absorbing layer maintaining the water absorbency, and
   a surface of the non-skin-side water-repellent region has a water contact angle of 80 degrees or greater and a water absorption rate of 10 seconds or greater.
2. The water repellency-imparted fiber article as set forth in clause 1, wherein both a portion to which the water repellency-imparting agent is attached and a portion to which the water repellency-imparting agent is not attached are present in the non-skin-side water-repellent region.
   3. The water repellency-imparted fiber article as set forth in clause 1 or 2, wherein in a 50-mm-square region arbitrarily selected from the non-skin-side water-repellent region, a percentage of the total area of a portion to which the water repellency-imparting agent is attached to the whole area of the 50-mm-square region is from 10% to 80%.
   4. The water repellency-imparted fiber article as set forth in any one of clauses 1 to 3, wherein a thickness of a portion to which the water repellency-imparting agent is attached, of the non-skin-side water-repellent region is from 5% to 60% of a thickness of the fiber article.
   5. The water repellency-imparted fiber article as set forth in any one of clauses 1 to 4, wherein a thickness of a portion to which the water repellency-imparting agent is attached, of the non-skin-side water-repellent region is from 50% to 60% of a thickness of the fiber article.
   6. The water repellency-imparted fiber article as set forth in any one of clauses 1 to 5, wherein an air permeance of a section where the non-skin-side water-repellent region is formed is from 2 seconds/100 ml to 30 seconds/100 ml.
   7. The water repellency-imparted fiber article as set forth in any one of clauses 1 to 6, wherein a bending resistance of a section where the non-skin-side water-repellent region is formed is 60 mm or less, and is equal to or lower than the bending resistance of a section of the water repellency-imparted fiber article where the non-skin-side water-repellent region is not formed, which is a water-repellent region non-formed portion.
8. The water repellency-imparted fiber article as set forth in any one of clauses 1 to 7, wherein an amount of the water repellency-imparting agent attached decreases from the non-skin-facing surface of the fiber article toward the skin-facing surface thereof.
   9. The water repellency-imparted fiber article as set forth in any one of clauses 1 to 8, wherein the water repellency-imparted fiber article has a skin-side water-repellent region on the skin-facing surface of the fiber article, the skin-side water-repellent region including a portion to which the water repellency-imparting agent is attached, and an amount of the water repellency-imparting agent attached in the skin-side water-repellent region is smaller than that in the non-skin-side water-repellent region.
   10. The water repellency-imparted fiber article as set forth in any one of clauses 1 to 9, wherein the non-skin-facing surface of the fiber article has an uneven structure, and the water repellency-imparting agent that forms the non-skin-side water-repellent region is attached to projections constituting the uneven structure.
   11. The water repellency-imparted fiber article as set forth in any one of clauses 1 to 9, wherein the water repellency-imparting agent is a composition containing a modified silicone.
   12. The water repellency-imparted fiber article as set forth in clause 11, wherein the water repellency-imparting agent contains a volatile solvent in which the modified silicone is soluble.
   13. The water repellency-imparted fiber article as set forth in clause 11 or 12, wherein the composition contains a water-soluble binder.
   14. The water repellency-imparted fiber article as set forth in any one of clauses 1 to 13, wherein, when the water repellency-imparted fiber article is washed by immersing the water repellency-imparted fiber article in a liquid containing 0.1 mass% of a commonly available detergent for clothing and ion exchanged water under stirring at a rotational speed of 350 rpm for 10 minutes, and then lightly squeezing the water repellency-imparted fiber article, and further immersing the water repellency-imparted fiber article in ion exchanged water under stirring at a rotational speed of 350 rpm for 10 minutes, an amount of the water repellency-imparting agent contained in the water repellency-imparted fiber article after washing is 20 mass% or less based on that before washing.
15. The water repellency-imparted fiber article as set forth in any one of clauses 1 to 14, wherein the fiber article contains a natural fiber and a synthetic fiber as constituent fibers thereof.
   16. The water repellency-imparted fiber article as set forth in any one of clauses 1 to 14, wherein the fiber article contains only a natural fiber as a constituent fiber thereof.
   17. The water repellency-imparted fiber article as set forth in any one of clauses 1 to 16, wherein the water repellency-imparted fiber article contains at least one selected from the group consisting of a deodorizing ingredient, an odor-removing ingredient, a fragrant ingredient, a antimicrobial agent, a bactericidal agent, and a refreshing agent.
   18. The water repellency-imparted fiber article as set forth in any one of clauses 1 to 17, wherein the fiber article is a piece of inner clothing.
   19. The water repellency-imparted fiber article as set forth in any one of clauses 1 to 17, wherein the fiber article a piece of sporting clothing.
   20. The water repellency-imparted fiber article as set forth in any one of clauses 1 to 17, wherein the fiber article is an incontinence pad.
21. A method for producing the water repellency-imparted fiber article as set forth in any one of clauses 1 to 20, the method comprising:
      applying a water repellency-imparting agent to a non-skin-facing surface of a fiber article having water absorbency in terms of a water absorption time of 30 seconds or less as measured in accordance with the dropping method of JIS L-1907.
   22. The method for producing the water repellency-imparted fiber article as set forth in clause 21, wherein after the water repellency-imparting agent is applied to the fiber article, the fiber article is dried under a condition that a temperature of the water repellency-imparting agent is 50°C or less.
   23. The method for producing the water repellency-imparted fiber article as set forth in clause 21 or 22, wherein the application of the water repellency-imparting agent to the fiber article is performed by spraying the water repellency-imparting agent.
   24. The method for producing the fiber article as set forth in any one of clauses 21 to 23, wherein the application of the water repellency-imparting agent to the fiber article is performed by spraying the water repellency-imparting agent with a pump spray-type application means in which the water repellency-imparting agent is put into a container equipped with a sprayer.
   25. The method for producing the fiber article as set forth in any one of clauses 21 to 24, wherein the application of the water repellency-imparting agent to the fiber article is performed by spraying the water repellency-imparting agent with an aerosol spray-type application means in which the water repellency-imparting agent and a propellant are put into a pressure-resistant container for aerosol spray.
   26. The method for producing the fiber article as set forth in any one of clauses 21 to 24, wherein the application of the water repellency-imparting agent to the fiber article is performed by spraying the water repellency-imparting agent with a manual spray-type application means in which the water repellency-imparting agent is put into a container equipped with a manual sprayer.
   27. The method for producing the fiber article as set forth in any one of clauses 21 to 23, wherein the application of the water repellency-imparting agent to the fiber article is performed with an application means including a stick-shaped solidified matter of the water repellency-imparting agent, the solidified matter being brought into contact with the fiber article as an object.
   28. The method for producing the fiber article as set forth in clause 21 or 22, wherein the application of the water repellency-imparting agent to the fiber article is performed with a roll-on application means in which the water repellency-imparting agent is put into a roll-on container.
   29. The method for producing the fiber article as set forth in any one of clauses 21 to 28, wherein an application means is used for applying the water repellency-imparting agent to the fiber article, and information about a usage method of the application means is provided such that the information is externally visible.

### Examples

Hereinafter, the present invention will be described in greater detail by way of Examples. However, the present invention is not limited thereto.

### Composition A as Water Repellency-Imparting Agent

A polyoxazoline modified silicone (silicone: 75 mass%, polyoxazoline: 25 mass%, see JP H5-25025A etc.) as an active of a water repellency-imparting agent was dissolved in an ethanol solvent at normal temperature to obtain a 20 mass% solution in ethanol, and the solution was then filled in a spray can together with a dimethyl ether/LPG (70/30) gas (weight ratio of ethanol solution/gas = 40/60) to obtain composition A as a water repellency-imparting agent in the form of a spray.

### Composition B as Water Repellency-Imparting Agent

A polyether modified silicone (trade name "SH-3775M" manufactured by Dow Corning Toray Co., Ltd.) serving as an active of a water repellency-imparting agent, a (vinyl methyl ether/butyl maleate) copolymer (trade name "Gantrez ES-425" available from Matsumoto Trading Co., Ltd.) serving as a water-soluble binder, and ethanol were mixed at a mass ratio of 10/10/80 and forcibly stirred. The resultant solution was filled in a spray can together with a dimethyl ether/LPG (70/30) gas (weight ratio of ethanol solution/gas = 40/60) to obtain composition B as a water repellency-imparting agent in the form of a spray. The dispersed state of composition B was unstable, and therefore, the spray can was shaken several time before use.

### Composition C as Water Repellency-Imparting Agent

A commercially-available spray-type water repellency-imparting agent (trade name "Scotchgard for clothes and fabric products" manufactured by 3M) was used as composition C as a water repellency-imparting agent as-is.

### Fiber Article I: Pre-treated Cotton Fabric

A raw fabric (oxford plain white cotton fabric, product number: 2012200003154, purchased at Yuzawaya) with a width of 60 cm, a length of 1 m, and a basis weight of 180 g/m² was provided. The raw fabric was pre-treated for the purpose of eliminating the effects of a process oil and the like. Specifically, the raw fabric was washed and rinsed, and dried for twenty-four hours in an environment of 23 ± 2°C / 50 ± 5% RH. After that, the raw fabric was ironed at a high temperature with use of an iron (NI-S33 manufactured by Panasonic Corporation) to remove wrinkles. The washing and rinsing were performed using a commercially-available washing machine (NW-500MX manufactured by Hitachi, Ltd.) and 30 g of a commercially-available detergent (Ultra Attack Neo manufactured by Kao Corporation) with 30 L of water, through the following process "washing for 4 minutes → a single rinse cycle → spinning for 5 minutes". The pre-treated cotton fabric thus obtained was used as fiber article I. Fiber article I had water absorbency in terms of a water absorption time of 30 seconds or less as measured in accordance with the dropping method of JIS L-1907.

### Example 1

Fiber article I (the pre-treated cotton fabric) having a 10 cm × 10 cm square shape in a plan view was placed on top of filter paper with one side thereof facing upward. A water repellency-imparting agent was applied onto the entire upper surface of fiber article I using a spray (with a pressure of 0.4 mPa) that was positioned at 25 cm above fiber article I for a spray duration time of 2 seconds. Then, fiber article I was allowed to stand for 30 minutes to dry. In this manner, a water-repellent region including a portion to which the water repellency-imparting agent was attached was formed over the entire surface on one side of fiber article I, to thereby obtain a water repellency-imparted fiber article. The areal weight of the water repellency-imparting agent in the obtained water repellency-imparted fiber article was 0.15 g/m² in terms of solid content. Composition A was used as the water repellency-imparting agent.

### Example 2

A water repellency-imparted fiber article was obtained in the same manner as in Example 1, except that the spray duration time for applying the water repellency-imparting agent with the spray was 10 seconds. The areal weight of the water repellency-imparting agent in the obtained water repellency-imparted fiber article was 1.10 g/m² in terms of solid content.

### Example 3

A water repellency-imparted fiber article was obtained in the same manner as in Example 1, except the following: composition B was used as the water repellency-imparting agent, fiber article I was dried (allowed to stand) for 5 minutes after spraying of the water repellency-imparting agent, and the following post-treatment (esterification treatment) was performed after drying. The areal weight of the water repellency-imparting agent in the obtained water repellency-imparted fiber article was 0.10 g/m² in terms of solid content.

### Post-Treatment after Application of Water Repellency-imparting Agent

The entire upper surface of the fiber article to which the water repellency-imparting agent was attached was covered with a press cloth (white broadcloth cotton fabric, product number: 2012200092448, purchased at Yuzawaya), and ironed over the press cloth at a low temperature with an iron (NI-S33 manufactured by Panasonic Corporation). Then, the fiber article was allowed to stand for 20 minutes. During this ironing at a low temperature, ironing was performed such that the temperature of the surface (upper surface) of the fiber article to which the water repellency-imparting agent was attached was 50°C.

### Comparative Example 1

A water repellency-imparted fiber article was obtained in the same manner as in Example 1, except that composition C was used as the water repellency-imparting agent. The areal weight of the water repellency-imparting agent in the obtained water repellency-imparted fiber article was 0.10 g/m² in terms of solid content.

### Comparative Example 2

Fiber article I (i.e., the pre-treated cotton fabric), which was an intermediate product for Example 1, was used as Comparative Example 2 as-is.

### Comparative Example 3

A water repellency-imparted fiber article was obtained in the same manner as in Example 1, except that the spray duration time for applying the water repellency-imparting agent (composition A) with the spray was 0.8 seconds. The areal weight of the water repellency-imparting agent in the obtained water repellency-imparted fiber article was 0.04 g/m² in terms of solid content.

### Comparative Example 4

A water repellency-imparted fiber article was obtained in the same manner as in Example 1, except that the spray duration time for applying the water repellency-imparting agent (composition A) with the spray was 3.5 seconds. The areal weight of the water repellency-imparting agent in the obtained water repellency-imparted fiber article was 0.29 g/m² in terms of solid content.

### Performance Evaluation I

On the fiber articles (water repellency-imparted fiber articles) of Examples and Comparative Examples, in which fiber article I was used as the base, the contact angle and the water repellency angle (sliding angle) on various portions, the water absorption rate, the occupancy ratio of portions to which the water repellency imparting agent was attached, the thickness ratio of the water repellency-imparting agent, the air permeance, the bending resistance, and other properties were determined in accordance with the above-described methods, and a tactile feel test, which will be described later, was also performed. Table 1 below shows the results. For a single sample, each of these determinations was performed five times at different observation positions, and an average of the five found values was used as a representative value for that sample. Moreover, the samples of Examples and Comparative Examples were subjected to a seepage test and a usage test, which will be described later. Table 2 below shows the results.

### 1. Tactile Feel Test

Five male panelists to touch with their fingers the entirety of each test sample having a 100 mm × 100 mm quadrangular shape in a plan view, and evaluated the overall tactile sensation and the softness at that time in accordance with the following evaluation criteria. The average of evaluation scores made by the five panelists was used as the evaluation result of the test sample. The higher the evaluation score was, the higher the evaluation was.

### Evaluation Criteria for Overall Tactile Sensation

1 point: Poor, 2 points: Slightly poor, 3 points: Fair, 4 points: Slightly good, 5 points: Good

### Evaluation Criteria for Softness

1 point: Hard, 2 points: Slightly hard, 3 points: Fair, 4 points: Slightly soft, 5 points: Soft

### 2. Seepage Test

Filter paper and a water-permeable sheet were laid one on top of the other in that order on a horizontal stage, and were allowed to stand for 2 hours in an environment at a temperature of 25°C. A fiber article to which no water repellency-imparting agent was applied, that is, the above-described pre-treated cotton fabric was used as the water-permeable sheet. Next, a fiber article (any one of Examples and Comparative Examples) as a test sample was laid on the water-permeable sheet. At that time, in the case where the test sample was fiber article in which the water repellency-imparting agent was applied to one side thereof, the test sample was laid on the water-permeable sheet such that the side to which the water repellency-imparting agent was applied faced the water-permeable sheet. Then, 1 mL of blue water was dropped onto the upper surface of the test sample with a dropper. The blue water was prepared by coloring distilled water with a coloring agent (Blue No. 1) (the content of the coloring agent in the blue water was 0.1 mass%). 5 minutes after the dropping of the blue water, the test sample was removed, and the remaining lower fabric and filter paper were each visually observed, and evaluated in accordance with the following evaluation criteria. In the present test, the test sample and the water-permeable sheet were regarded as a piece of inner clothing such as underwear, and a piece of outer clothing, respectively, and the seepage of a liquid (blue water) from the test sample side to the water-permeable-sheet side was evaluated.

### Evaluation Criteria for Seepage

Very good: Any portion to which the blue water was attached were not present both in the water-permeable sheet and in the filter paper.

Good: A portion to which the blue water was attached, the portion having a dot-like shape in a plan view and a maximum diameter of 5 mm or less, was present in the water-permeable sheet, but any portion to which the blue water was attached were not present in the filter paper.

Poor: A relatively large portion (so-called stain) to which the blue water was attached and which had a maximum diameter of greater than 20 mm was present in the water-permeable sheet, but any portion to which the blue water were not attached was present in the filter paper.

Very poor: A portion to which the blue water was attached and which at least had a diameter of 2 mm or greater was observed in the filter paper; or a relatively large portion (so-called stain) to which the blue water was attached and which had a maximum diameter of greater than 40 mm was present in the water-permeable sheet, and a portion to which the blue water was attached was present in the filter paper.

On the sample of Example 1, an additional seepage test was performed, in addition to the above-described seepage test. In the additional seepage test, the same procedure as of the above-described seepage test was performed, except that the water-permeable sheet and the evaluation sample were replaced by each other. In this additional seepage test, the water-permeable sheet and the test sample were regarded as a piece of inner clothing such as underwear and a piece of outer clothing, respectively, and the seepage of a liquid (blue water) from the water-permeable-sheet side to the test sample side was evaluated. In Table 2 below, the above-described seepage test is indicated by "Suitability for inner clothing", the additional seepage test is indicated by "Suitability for outer clothing", and the two tests are thereby differentiated from each other.

### 3. Usage Test (Pants)

Two men who were conscious of the occurrence of so-called "after-dribble" at least once a day served as panelists. Each of the two panelists were given 100% cotton underpants (inner clothing) of a type he usually uses, as well as compositions A to C (each in the form of a spray) as the water repellency-imparting agent. The panelists were instructed to spray any one of the compositions on the non-skin-facing surface side of the underpants, dry the underpants for one day, then wear the resulting underpants and trousers (jeans), which are outer clothing, on the underpants, and go about their lives as usual in that state. The test period was two days for each water repellency-imparting agent, and if the underpants were soiled due to after-dribble or the like, the underpants were replaced every time with a new pair of underpants on which the water repellency-imparting agent was sprayed. During the test period, the panelists made sensory evaluations of (1-1) the number of times urine seeped into the trousers due to after-dribble, (1-2) the result of the occurrence of after-dribble, (1-3) the overall evaluation of the underpants, and (1-4) the wearing comfort of the underpants. The average of the evaluations made by the two panelists was used as the evaluation result with respect to the test sample.

With regard to item (1-1), when after-dribble occurred in a panelist, the panelist visually observed the trousers from outside. If a urine stain in the trousers was found, the number of times urine seeped into the trousers was one, and if no urine stain was found in the trousers, the number of times urine seeped into the trousers was zero. It can be determined that as the number of times urine seeped into the trousers was smaller, the seepage-suppressing effect of the underpants to which the water repellency-imparting agent was attached (water repellency-imparted fiber article) was more significant and thus highly rated.

With regard to item (1-2), the result of the occurrence of after-dribble refers to matters that the panelist actually perceived when after-dribble occurred in that panelist, and if the panelist did not perceive urine seeping into the trousers side, the "seepage into the trousers was prevented", which was highly rated. Otherwise, the panelist reported the specific result, and for example, the panelist reported as follows: urine passed through the underpants and transferred to the trousers side; urine flowed out from the bottom of the underpants; and so on.

With regard to item (1-3), the panelists made overall evaluation of the underpants, in comparison with the same type of underpants that they usually wear, on a five-point scale with consideration given to the relief and the like when wearing the underpants. As the evaluation score was higher, the test sample was highly rated.

1 point: Poor, 2 points: Slightly poor, 3 points: Equivalent, 4 points: Slightly good, 5 points: Good

With regard to item (1-4), the panelists evaluated the stiff feel (hardness), in comparison with the same type of underpants that they usually wear, on the following five-point scale. As the evaluation score was higher, the test sample was more highly rated.

1 point: Feel stiff, 2 points: Feel slightly stiff, 3 points: Neither, 4 points: Feel slightly not stiff, 5 points: Feel not stiff

**Table 1**

| | | Examples | | | Comparative Examples | | | |
|---|---|---|---|---|---|---|---|---|
| | | 1 | 2 | 3 | 1 | 2 | 3 | 4 |
| Water repellency-imparting agent | | A | A | B | C | - | A | A |
| Amount of water repellency-imparting agent attached (g/m²) | | 0.15 | 1.10 | 0.10 | 0.10 | 0 | 0.04 | 0.29 |
| Occupancy ratio of portion to which water repellency-imparting agent is attached (%) | | 45 | 68 | 45 | 89 | 0 | 9 | 88 |
| Thickness ratio of water repellency-imparting agent (%) | | 60 | 55 | 50 | 95 | 0 | 38 | 67 |
| Air permeance (sec.) | | 17 | 22 | 17 | 32 | 15 | 16 | 35 |
| Water contact angle on surface to which water repellency-imparting agent is applied (°) | | 90 | 98 | 85 | 100 | N.D. *1 | N.D. *1 | 100 |
| Water contact angle on surface to which water repellency-imparting agent is not applied (°) | | N.D.*1 | 60 | N.D. *1 | 100 | N.D. *1 | N.D. *1 | 90 |
| Water repellency angle on surface to which water repellency-imparting agent is applied (°) | | 30 | 10 | 40 | 10 | 80 | 80 | 10 |
| Water repellency angle on surface to which water repellency-imparting agent is not applied (°) | | N.D. | 60 | N.D. | 10 | 80 | 80 | 15 |
| Water absorption rate of surface to which water repellency-imparting agent is applied (sec.) | | 50 | 60 sec. ≤ | 40 | 60 sec. ≤ | Instant*2 | Instant*2 | 60 sec. ≤ |
| Water absorption rate of surface to which water repellency-imparting agent is not applied (sec.) | | Instant*2 | 7 | Instant*2 | 60 sec. ≤ | Instant*2 | Instant*2 | 40 |
| Bending resistance (mm) | | 40 | 50 | 55 | 65 | 50 | 50 | 35 |
| Tactile feel test | Overall tactile sensation (points) | 5.0 | 5.0 | 4.2 | 2.4 | 4.4 | 5.0 | 2.0 |
| | Softness (points) | 5.0 | 5.0 | 4.0 | 2.4 | 4.4 | 5.0 | 5.0 |

| | | | | | | | | |
|---|---|---|---|---|---|---|---|---|
| *1: The liquid drop used in determination was instantly absorbed, and thus determination was impossible. *2: The water absorption rate was too fast to be determined, and thus determination was impossible. | | | | | | | | |

**Table 2**

| | | Examples | | | Comparative Examples | | | |
|---|---|---|---|---|---|---|---|---|
| | | 1 | 2 | 3 | 1 | 2 | 3 | 4 |
| Water repellency-imparting agent | | A | A | B | C | - | A | A |
| Amount of water repellency-imparting agent attached (g/m²) | | 0.15 | 1.10 | 0.10 | 0.10 | 0 | 0.04 | 0.29 |
| Occupancy ratio of portion to which water repellency-imparting agent is attached (%) | | 45 | 68 | 45 | 89 | 0 | 9 | 88 |
| Thickness ratio of water repellency-imparting agent (%) | | 60 | 55 | 50 | 95 | 0 | 38 | 67 |
| Air permeance (sec.) | | 17 | 22 | 17 | 32 | 15 | 16 | 35 |
| Water contact angle on surface to which water repellency-imparting agent is applied (°) | | 90 | 98 | 85 | 100 | N.D. *1 | N.D. *1 | 100 |
| Water contact angle on surface to which water repellency-imparting agent is not applied (°) | | N.D. *1 | 60 | N.D. *1 | 100 | N.D. *1 | N.D. *1 | 90 |
| Water repellency angle on surface to which water repellency-imparting agent is applied (°) | | 30 | 10 | 40 | 10 | 80 | 80 | 10 |
| Water repellency angle on surface to which water repellency-imparting agent is not applied (°) | | N.D. | 60 | N.D. | 10 | 80 | 80 | 15 |
| Water absorption rate of surface to which water repellency-imparting agent is applied (sec.) | | 50 | 60 sec. ≤ | 40 | 60 sec. ≤ | Instant*2 | Instant*2 | 60 sec. ≤ |
| Water absorption rate of surface to which water repellency-imparting agent is not applied (sec.) | | Instant*2 | 7 | Instant*2 | 60 sec. ≤ | Instant*2 | Instant*2 | 40 |
| Bending resistance (mm) | | 40 | 50 | 55 | 65 | 50 | 50 | 35 |
| Seepage test | Suitability for inner clothing | Very good | Very good | Very good | Very poor | Very poor | Very poor | Very poor |
| | Suitability for outer clothing | Very good | - | - | - | - | - | - |
| Usage test | Number of times urine seeped into trousers (times) | 0 | 0 | 0 | 2 | 2 | 2 | 2 |
| | Result of occurrence of after-dribble | Seepage of urine into trousers was prevented. | Seepage of urine into trousers was prevented. | Seepage of urine into trousers was prevented. | Every time after-dribble occurred, urine flowed inside underpants and seeped into trousers from bottom of underpants. | Every time after-dribble occurred, urine passed through underpants and seeped into trousers. | Every time after-dribble occurred, urine passed through underpants and seeped into trousers. | Every time after-dribble occurred, urine flowed inside underpants and seeped into trousers from bottom of underpants. |
| | Overall evaluation (points) | 5.0 | 5.0 | 4.0 | 2.5 | 2.0 | 2.0 | 2.0 |
| | Wearing comfort (points) | 5.0 | 5.0 | 4.0 | 2.5 | 4.0 | 2.0 | 2.0 |

| | | | | | | | | |
|---|---|---|---|---|---|---|---|---|
| *1: The liquid drop used in determination was instantly absorbed, and thus determination was impossible. *2: The water absorption rate was too fast to be determined, and thus determination was impossible. | | | | | | | | |

As shown in Tables 1 and 2, in all of the fiber articles of Examples, which were water repellency-imparted fiber articles in which the water repellency-imparting agent was applied to the entire surface on one side thereof, the surface to which the water repellency-imparting agent was applied (surface of water-repellent region) had a water contact angle of 80 degrees or greater (water repellency angle of 40 degrees or less) and a water absorption rate of 10 seconds or greater, and also, the surface to which the water repellency-imparting agent was not applied and which was located on the opposite side to the surface to which the water repellency-imparting agent was applied exhibited high water absorbency such that the water contact angle was 60 degrees or less, or such that the water repellency angle was 50 degrees or greater, or such that the contact angle was not able to be measured. Thus, it can be determined that all the fiber articles of Examples had a water absorbing layer maintaining the "water absorbency in terms of a water absorption time of 30 seconds or less as measured in accordance with the dropping method of JIS L-1907", the water absorbing layer being located on the side that was closer to the skin of the user than the non-skin-side water-repellent region including the portion to which the water repellency-imparting agent composition was attached. Therefore, all the fiber articles of Examples exhibited the excellent effect of preventing of bodily fluid seepage, compared with Comparative Examples. Moreover, from the favorable results of the usage test, it can be seen that the fiber articles of Examples can be effective in addressing after-dribble when actually used for underpants. Furthermore, even though the fiber articles of Example had water-repellency, those fiber articles had high air permeance and were less likely to cause dampness, compared with Comparative Examples 1 and 4, which were the fiber articles that had water-repellency as well. In addition, the results of the tactile feel test of Examples were equal to or better than those of Comparative Example 2, which was the fiber article that was not made water-repellent. Therefore, it is clear that, according to the present invention exemplified by Examples, bodily fluid seepage, specifically, urine seepage due to after-dribble, or the like can be effectively prevented without impairing the texture of the fiber article.

Unlike Comparative Example 2, the fiber article of Comparative Example 1 had water-repellency through the application of the water repellency-imparting agent; however, the surface thereof to which the water repellency-imparting agent was not applied had a water contact angle of 60 degrees or greater (water repellency angle of less than 50 degrees), and it is therefore considered that this fiber article did not have a water absorbing layer that maintained the above-described water absorbency, on the side that was closer to the skin of the user than the non-skin-side water-repellent region including the portion to which the water repellency-imparting agent was attached. In other words, this fiber article was made water-repellent over the entire area in the thickness direction thereof, and thus, the intrinsic water absorbency thereof was significantly impaired. It is inferred that, for this reason, the results of the seepage test were poor, and that, in the usage test, urine flowed down on the inner side of the underwear when after-dribble occurred, which resulted in an extremely poor usage feel and failure in prevention of seepage.

The fiber article of Comparative Example 3 had water-repellency through the application of the water repellency-imparting agent. However, the thickness ratio of the water repellency-imparting agent was not within a suitable range, that is, not within a range of 50 to 60%, which was the range of Examples 1 to 3, and specifically, the thickness ratio of the water repellency-imparting agent was 38%, which was excessively low. Accordingly, the function of the non-skin-side water-repellent region as a barrier layer was insufficient, and it is inferred that, for this reason, the results of the seepage test and the usage test were poor. On the other hand, the fiber article of Comparative Example 4 had a thickness ratio of the water repellency-imparting agent of 67%, which was excessively high. Accordingly, as is the case with Comparative Example 1, the intrinsic water absorbency of the underwear, which was located on the skin-facing surface side, was not sufficiently used, and it is thought that, for this reason, every time after-dribble occurred, urine flowed down on the inner side of the underwear to cause seepage and impair the usage feel significantly. Moreover, due to the effect of the excessive amount of water repellency-imparting agent, the numerical value of the air permeance was high, which was detrimental to the air permeability, and, in addition, the difference in the numerical value of the bending resistance from that of Comparative Example 2, to which the water repellency-imparting agent composition was not applied, was considerable, which means that the fiber article was excessively flexible and was also sticky to the touch. It is inferred that, for this reason, the result with respect to the overall tactile sensation was poor. Composition A as a water repellency-imparting agent used in Comparative Example 4 was a type of modified silicone-containing water repellency-imparting agent which has been described above, and had a property such that the flexibility of the section to which the agent is attached more increased (the stiffness of that section more decreased) as the amount of the agent attached was larger.

### Fiber Article II: Inner Shirt

A commercially-available 100% cotton inner shirt (YG V neck from Gunze Limited, product number: 31YV0015N) was used as fiber article II as-is. Fiber article II had water absorbency in terms of a water absorption time of 30 seconds or less as measured in accordance with the dropping method of JIS L-1907.

### Example 4

Fiber article II (inner shirt) was placed on filter paper with the outer surface (non-skin-facing surface) on the back side thereof facing upward. A water repellency-imparting agent was applied onto the central portion of the back-side outer surface of fiber article II using a spray (with a pressure of 0.4 mPa) that was positioned at 25 cm above fiber article II. Then, fiber article II was allowed to stand to dry. During spraying, the water repellency-imparting agent was sprayed over a region having a rectangular shape in a plan view with a length of 20 cm and a width of 30 cn. The water repellency-imparting agent was sprayed for 10 seconds, and was uniformly sprayed over the rectangular region entirely so that the water repellency-imparting agent was prevented from accumulating at one portion on fiber article II. The drying after spraying was performed by allowing fiber article II to which the water repellency-imparting agent was applied to stand for twenty-four hours in an environment at room temperature

(environment at 23 ± 2°C / 50 ± 5% RH). The areal weight of the water repellency-imparting agent in the obtained water repellency-imparted fiber article was 0.10 g/m² in terms of solid content. Composition A was used as the water repellency-imparting agent.

### Comparative Example 5

A water repellency-imparted fiber article was obtained in the same manner as in Example 4, except that composition C was used as the water repellency-imparting agent. The areal weight of the water repellency-imparting agent in the obtained water repellency-imparted fiber article was 0.10 g/m² in terms of solid content.

### Comparative Example 6

Fiber article II, which was an intermediate product for Example 4, that is, the commercially-available cotton shirt was used as Comparative Example 6 as-is.

### Performance Evaluation II

On the fiber articles (water repellency-imparted fiber articles), in which fiber article II was used as the base, of Example and Comparative Examples, the contact angle and the water repellency angle (sliding angle) on various portions, the water absorption rate, the occupancy ratio of portions to which the water repellency imparting agent was attached, the thickness ratio of the water repellency-imparting agent, the air permeance, the bending resistance, and other properties were determined in accordance with the above-described methods, and the above-described tactile feel test was also performed. Table 3 below shows the results. For a single sample, each of these determinations was performed five times at different observation positions, and an average of the five found values was used as a representative value for that sample. Moreover, the samples of Example and Comparative Examples were subjected to the above-described seepage test, and a usage test below. Table 4 below shows the results.

### Usage Test (Inner Shirt)

Each of two male panelists was given following items: six, commercially-available 100% cotton inner shirts (YG V neck from Gunze Limited, product number: 31YV0015N) as pieces of inner clothing, the inner shirts being the right size for the panelist; compositions A and C (each in the form of a spray) as the water repellency-imparting agent; and two commercially-available polo shirts (UNIQLO dry pique polo shirts, short-sleeve, gray, product number: 180719, 72% cotton, 28% polyester) as pieces of outer clothing to be worn directly on the inner shirts, the polo shirts being the right size for the panelist. The panelists wore the inner shirt after spraying a water repellency-imparting agent onto the central portion of the back-side outer surface of the inner shirt and drying the inner shirt. With respect to spraying and drying, the panelists were instructed to spray the water repellency-imparting agent onto a region having a rectangular shape in a plan view with a length of 20 cm and a width of 30 cm for a spraying time of 10 seconds. Furthermore, the panelists were instructed to spray the water repellency-imparting agent uniformly over the rectangular region entirely so that the water repellency-imparting agent was prevented from accumulating at one portion on the inner shirt, and also instructed to dry the inner shirt after spraying at room temperature for twenty-four hours before wearing. Furthermore, the panelists were instructed to wear the outer clothing (polo shirt) directly on the sprayed and dried inner shirt, and go about their lives as usual in this state. The test period was two days for each water repellency-imparting agent, and the outer clothing was washed every day and worn. During the test period, the panelists made sensory evaluations of (2-1) the number of times sweat seeped into the back of the outer clothing due to sweating, (2-2) the result of sweat staining, (2-3) the overall evaluation of the inner shirts, and (2-4) the wearing comfort of the inner shirts. The average of the evaluations made by the two panelists was used as the evaluation result with respect to the relevant test sample.

With regard to item (2-1), the panelists went about their lives as usual, and visually observed their backs in a mirror at about two o'clock p.m. every day. If a sweat stain in the outer clothing was found, the number of times sweat seepage occurred was one, and if no sweat stain in the outer clothing was found, the number of times sweat seepage occurred was zero. It can be determined that, as the number of sweat seepage occurred was smaller, the seepage-suppressing effect of the inner shirt (water repellency-imparted fiber article) to which the water repellency-imparting agent was attached was more significant and thus highly rated.

With regard to item (2-2), the result of sweat staining refers to the matters that the panelist felt when observing their backs to evaluate item (2-1), and if seepage into the outer clothing did not occur, the "seepage into the outer clothing was prevented", which was highly rated. Otherwise, the panelist reported the specific result, and for example, the panelist reported as follows: sweat passed through the inner shirt and transferred to the outer clothing side; sweat dripped down the region to which the water repellency-imparting agent was sprayed, causing a statin in a portion below that region or causing dampness; and so on.

With regard to item (2-3), the panelists made overall evaluation of the inner shirts, in comparison with the same type of inner shirts that they usually wear, on a five-point scale with consideration given to the relief and the like when wearing the inner shirts. As the evaluation score was higher, the test sample was highly rated.

1 point: Poor, 2 points: Slightly poor, 3 points: Equivalent, 4 points: Slightly good, 5 points: Good

With regard to item (2-4), the panelists evaluated the wearing comfort in terms of a damp feel, stickiness, a stiff feel (hardness), and the like, in comparison with the same type of inner shirts that they usually wear, on the following five-point scale. As the evaluation score was higher, the test sample was more highly rated

1 point: Poor, 2 points: Slightly poor, 3 points: Equivalent, 4 points: Slightly good, 5 points: Good

**Table 3**

| | | Example | Comparative Examples | |
|---|---|---|---|---|
| | | 4 | 5 | 6 |
| Water repellency-imparting agent | | A | C | - |
| Amount of water repellency-imparting agent attached (g/m²) | | 0.10 | 0.10 | 0 |
| Occupancy ratio of portion to which water repellency-imparting agent is attached (%) | | 45 | 89 | 0 |
| Thickness ratio of water repellency-imparting agent (%) | | 60 | 95 | 0 |
| Air permeance (sec.) | | 17 | 32 | 15 |
| Water contact angle on surface to which water repellency-imparting agent is applied (°) | | 90 | 100 | N.D. *1 |
| Water contact angle on surface to which water repellency-imparting agent is not applied (°) | | N.D.*1 | 100 | N.D.*1 |
| Water repellency angle on surface to which water repellency-imparting agent is applied (°) | | 30 | 10 | 80 |
| Water repellency angle on surface to which water repellency-imparting agent is not applied (°) | | N.D. | 10 | 80 |
| Water absorption rate of surface to which water repellency-imparting agent is applied (sec.) | | 50 | 60 sec. ≤ | Instant*2 |
| Water absorption rate of surface to which water repellency-imparting agent is not applied (sec.) | | Instant*2 | 60 sec. ≤ | Instant*2 |
| Bending resistance (mm) | | 40 | 65 | 50 |
| Tactile feel test | Overall tactile sensation (points) | 5.0 | 2.4 | 4.4 |
| | Softness (points) | 5.0 | 2.4 | 4.4 |

| | | | | |
|---|---|---|---|---|
| *1: The liquid drop used in determination was instantly absorbed, and thus determination was impossible. *2: The water absorption rate was too fast to be determined, and thus determination was impossible. | | | | |

**Table 4**

| | | Example | Comparative Examples | |
|---|---|---|---|---|
| | | 4 | 5 | 6 |
| Water repellency-imparting agent | | A | C | - |
| Amount of water repellency-imparting agent attached (g/m²) | | 0.10 | 0.10 | 0 |
| Occupancy ratio of portion to which water repellency-imparting agent is attached (%) | | 45 | 89 | 0 |
| Thickness ratio of water repellency-imparting agent (%) | | 60 | 95 | 0 |
| Air permeance (sec.) | | 17 | 32 | 15 |
| Water contact angle on surface to which water repellency-imparting agent is applied (°) | | 90 | 100 | N.D. *1 |
| Water contact angle on surface to which water repellency-imparting agent is not applied (°) | | N.D. *1 | 100 | N.D. *1 |
| Water repellency angle on surface to which water repellency-imparting agent is applied (°) | | 30 | 10 | 80 |
| Water repellency angle on surface to which water repellency-imparting agent is not applied (°) | | N.D. | 10 | 80 |
| Water absorption rate of surface to which water repellency-imparting agent is applied (sec.) | | 50 | 60 sec. ≤ | Instant*2 |
| Water absorption rate of surface to which water repellency-imparting agent is not applied (sec.) | | Instant*2 | 60 sec. ≤ | Instant*2 |
| Bending resistance (mm) | | 40 | 65 | 50 |
| Seepage test | Suitability for inner clothing | Very good | Very poor | Very poor |
| | Suitability for outer clothing | Very good | - | - |
| Usage test | Number of times seepage occurred (times) | 0 | 2 | 2 |
| | Result of sweat staining | Sweat staining of the back of polo shirt was prevented. | Dampness and sweating. Sweat flowed down on the back and left a stain below. | When looking at in daytime, round sweat stain was formed on the back. |
| | Overall evaluation (points) | 5.0 | 2.0 | 2.0 |
| | Wearing comfort (points) | 5.0 | 1.5 | 3.5 |

| | | | | |
|---|---|---|---|---|
| *1: The liquid drop used in determination was instantly absorbed, and thus determination was impossible. *2: The water absorption rate was too fast to be determined, and thus determination was impossible. | | | | |

As shown in Tables 3 and 4, in the fiber article of Example 4, which was a water repellency-imparted fiber article in which the water repellency-imparting agent was applied to the entire surface on one side thereof, the surface to which the water repellency-imparting agent was applied (surface of the water-repellent region) had a water contact angle of 80 degrees or greater (water repellency angle of 40 degrees or less) and a water absorption rate of 10 seconds or greater, and also, the surface of the fiber article to which the water repellency-imparting agent was not applied and which was located on the opposite side to the surface to which the water repellency-imparting agent was applied exhibited high water absorbency such that the water contact angle thereon was 60 degrees or less, or such that the water repellency angle thereon was 50 degrees or greater, or such that the contact angle was not able to be measured. Thus, it can be determined that the fiber article of Example 4 had a water absorbing layer maintaining the "water absorbency in terms of a water absorption time of 30 seconds or less as measured in accordance with the dropping method of JIS L-1907", the water absorbing layer being located on the side that was closer to the skin of the user than the non-skin-side water-repellent region including the portion to which the water repellency-imparting agent was attached. Therefore, the fiber article of Example 4 exhibited the excellent effect of preventing bodily fluid seepage, compared with Comparative Examples 5 and 6. Also, from the favorable results of the usage test, it can be seen that the fiber article of Example 4 can be effective in addressing sweat staining when actually used for an inner shirt. Furthermore, even though the fiber article of Example 4 had water-repellency, this fiber article had high air permeance and was less likely to cause dampness, compared with Comparative Example 5, which was the fiber article that had water-repellency as well. Also, the results of the tactile feel test of Example 4 were equal to or better than those of Comparative Example 6, which was the fiber article that was not made water-repellent. It is therefore clear that, according to the present invention exemplified by Example 4, bodily fluid seepage, specifically, sweat staining can be effectively prevented without impairing the texture of the fiber article.

Although the test results shown in Tables 3 and 4 were obtained in the case where the water repellency-imparting agent was applied to the inner shirts (inner clothing), the effect of preventing sweat staining similar to that in the case where the water repellency-imparting agent was applied to the inner shirts can also be obtained in the case where the water repellency-imparting agent is applied to, instead of the inner shirts, a polo shirt (outer clothing) that is worn on an inner shirt, in particular, in the case where the water repellency-imparting agent is applied to the skin-facing surface (inner surface) side of that polo shirt.

### Industrial Applicability

According to the present invention, a water repellency-imparted fiber article is provided which can effectively prevent a bodily fluid excreted from the body from seeping into clothing and thus becoming externally visible.

The water repellency-imparted fiber article of the present invention basically has a simple configuration in which a water repellency-imparting agent is just attached to a fiber article, which is the base of the water repellency-imparted fiber article, and the appearance and the basic performance of the water repellency-imparted fiber article are substantially unchanged from those intrinsic to the fiber article. Therefore, even though a measure to prevent bodily fluid seepage is taken in the water repellency-imparted fiber article of the present invention, this is not noticed by other people. Moreover, the water repellency-imparted fiber article also exhibits excellent texture, usage feel, and the like as a fiber article.

## Claims

1. A water repellency-imparted fiber article, comprising:
a fiber article having a skin-facing surface to be disposed on a side that is relatively close to the skin of a user during use and a non-skin-facing surface to be disposed on a side that is relatively away from the skin of the user, the fiber article containing a water-absorbent fiber and having water absorbency in terms of a water absorption time of 30 seconds or less as measured in accordance with the dropping method of JIS L-1907; and
a water repellency-imparting agent attached to the fiber article,
wherein the water repellency-imparted fiber article has a non-skin-side water-repellent region on the non-skin-facing surface of the fiber article, the non-skin-side water-repellent region including a portion to which the water repellency-imparting agent is attached,
the water repellency-imparted fiber article has a water absorbing layer in a section of the fiber article that overlaps the non-skin-side water-repellent region in a plan view and on a side that is closer to the skin of the user than the non-skin-side water-repellent region, the water absorbing layer maintaining the water absorbency, and
a surface of the non-skin-side water-repellent region has a water contact angle of 80 degrees or greater and a water absorption rate of 10 seconds or greater.

2. The water repellency-imparted fiber article according to claim 1, wherein both a portion to which the water repellency-imparting agent is attached and a portion to which the water repellency-imparting agent is not attached are present in the non-skin-side water-repellent region.

3. The water repellency-imparted fiber article according to claim 1 or 2, wherein in a 50-mm-square region arbitrarily selected from the non-skin-side water-repellent region, a percentage of the total area of a portion to which the water repellency-imparting agent is attached to the whole area of the 50-mm-square region is from 10% to 80%.

4. The water repellency-imparted fiber article according to any one of claims 1 to 3, wherein a thickness of a portion to which the water repellency-imparting agent is attached, of the non-skin-side water-repellent region is from 5% to 60% of a thickness of the fiber article.

5. The water repellency-imparted fiber article according to any one of claims 1 to 4, wherein a thickness of a portion to which the water repellency-imparting agent is attached, of the non-skin-side water-repellent region is from 50% to 60% of a thickness of the fiber article.

6. The water repellency-imparted fiber article according to any one of claims 1 to 5, wherein an air permeance of a section where the non-skin-side water-repellent region is formed is from 2 seconds/100 ml to 30 seconds/100 ml.

7. The water repellency-imparted fiber article according to any one of claims 1 to 6, wherein a bending resistance of a section where the non-skin-side water-repellent region is formed is 60 mm or less, and is equal to or lower than the bending resistance of a section of the water repellency-imparted fiber article where the non-skin-side water-repellent region is not formed, which is a water-repellent region non-formed portion.

8. The water repellency-imparted fiber article according to any one of claims 1 to 7, wherein an amount of the water repellency-imparting agent attached decreases from the non-skin-facing surface of the fiber article toward the skin-facing surface thereof.

9. The water repellency-imparted fiber article according to any one of claims 1 to 8, wherein the water repellency-imparted fiber article has a skin-side water-repellent region on the skin-facing surface of the fiber article, the skin-side water-repellent region including a portion to which the water repellency-imparting agent is attached, and an amount of the water repellency-imparting agent attached in the skin-side water-repellent region is smaller than that in the non-skin-side water-repellent region.

10. The water repellency-imparted fiber article according to any one of claims 1 to 9, wherein the non-skin-facing surface of the fiber article has an uneven structure, and the water repellency-imparting agent that forms the non-skin-side water-repellent region is attached to projections constituting the uneven structure.

11. The water repellency-imparted fiber article according to any one of claims 1 to 9, wherein the water repellency-imparting agent is a composition containing a modified silicone.

12. The water repellency-imparted fiber article according to claim 11, wherein the water repellency-imparting agent contains a volatile solvent in which the modified silicone is soluble.

13. The water repellency-imparted fiber article according to claim 11 or 12, wherein the composition contains a water-soluble binder.

14. The water repellency-imparted fiber article according to any one of claims 1 to 13, wherein, when the water repellency-imparted fiber article is washed by immersing the water repellency-imparted fiber article in a liquid containing 0.1 mass% of a commonly available detergent for clothing and ion exchanged water under stirring at a rotational speed of 350 rpm for 10 minutes, and then lightly squeezing the water repellency-imparted fiber article, and further immersing the water repellency-imparted fiber article in ion exchanged water under stirring at a rotational speed of 350 rpm for 10 minutes, an amount of the water repellency-imparting agent contained in the water repellency-imparted fiber article after washing is 20 mass% or less based on that before washing.

15. The water repellency-imparted fiber article according to any one of claims 1 to 14, wherein the fiber article contains a natural fiber and a synthetic fiber as constituent fibers thereof.

16. The water repellency-imparted fiber article according to any one of claims 1 to 14, wherein the fiber article contains only a natural fiber as a constituent fiber thereof.

17. The water repellency-imparted fiber article according to any one of claims 1 to 16, wherein the water repellency-imparted fiber article contains at least one selected from the group consisting of a deodorizing ingredient, an odor-removing ingredient, a fragrant ingredient, a antimicrobial agent, a bactericidal agent, and a refreshing agent.

18. The water repellency-imparted fiber article according to any one of claims 1 to 17, wherein the fiber article is a piece of inner clothing.

19. The water repellency-imparted fiber article according to any one of claims 1 to 17, wherein the fiber article a piece of sporting clothing.

20. The water repellency-imparted fiber article according to any one of claims 1 to 17, wherein the fiber article is an incontinence pad.

21. A method for producing the water repellency-imparted fiber article according to any one of claims 1 to 20, the method comprising:
applying a water repellency-imparting agent to a non-skin-facing surface of a fiber article having water absorbency in terms of a water absorption time of 30 seconds or less as measured in accordance with the dropping method of JIS L-1907.

22. The method for producing the water repellency-imparted fiber article according to claim 21, wherein after the water repellency-imparting agent is applied to the fiber article, the fiber article is dried under a condition that a temperature of the water repellency-imparting agent is 50°C or less.

23. The method for producing the water repellency-imparted fiber article according to claim 21 or 22, wherein the application of the water repellency-imparting agent to the fiber article is performed by spraying the water repellency-imparting agent.

24. The method for producing the fiber article according to any one of claims 21 to 23, wherein the application of the water repellency-imparting agent to the fiber article is performed by spraying the water repellency-imparting agent with a pump spray-type application means in which the water repellency-imparting agent is put into a container equipped with a sprayer.

25. The method for producing the fiber article according to any one of claims 21 to 24, wherein the application of the water repellency-imparting agent to the fiber article is performed by spraying the water repellency-imparting agent with an aerosol spray-type application means in which the water repellency-imparting agent and a propellant are put into a pressure-resistant container for aerosol spray.

26. The method for producing the fiber article according to any one of claims 21 to 24, wherein the application of the water repellency-imparting agent to the fiber article is performed by spraying the water repellency-imparting agent with a manual spray-type application means in which the water repellency-imparting agent is put into a container equipped with a manual sprayer.

27. The method for producing the fiber article according to any one of claims 21 to 23, wherein the application of the water repellency-imparting agent to the fiber article is performed with an application means including a stick-shaped solidified matter of the water repellency-imparting agent, the solidified matter being brought into contact with the fiber article as an object.

28. The method for producing the fiber article according to claim 21 or 22, wherein the application of the water repellency-imparting agent to the fiber article is performed with a roll-on application means in which the water repellency-imparting agent is put into a roll-on container.

29. The method for producing the fiber article according to any one of claims 21 to 28, wherein an application means is used for applying the water repellency-imparting agent to the fiber article, and information about a usage method of the application means is provided such that the information is externally visible.
